# EUROPEAN PATENT SPECIFICATION

(11) **EP 3 500 248 B1**
(45) Date of publication and mention of the grant of the patent: **02.12.2026**
(21) Application number: 17842255.6
(22) Date of filing: 19.08.2017
(51) Int. Cl.: A61K 9/20, B29C 64/106, B33Y 80/00, B33Y 70/00, B33Y 40/00, B33Y 30/00, B33Y 10/00, A61K 31/167, A61K 31/192

(54) **METHODS AND APPARATUSES FOR THE MANUFACTURE OF FIBROUS DOSAGE FORMS**
VERFAHREN UND VORRICHTUNGEN ZUR HERSTELLUNG VON FASERIGEN DARREICHUNGSFORMEN
PROCÉDÉS ET APPAREILS DE FABRICATION DE FORMES POSOLOGIQUES FIBREUSES

(30) Priority: 19.08.2016 US 201662377068 P; 26.10.2016 WO PCT/US2016/058935; 14.01.2017 US 201762446431 P; 08.03.2017 US 201762468888 P; 09.04.2017 US 201715482776; 11.07.2017 WO PCT/US2017/041609
(43) Date of publication of application: 26.06.2019
(73) Proprietor: Blaesi, Aron H., 7078 Lenzerheide (CH)
(72) Inventor: BLAESI, Aron H., 7078 Lenzerheide (CH); SAKA, Nannaji, Cambridge, MA 02139 (US)
(86) International application number: PCT/US2017/047703
(87) International publication number: WO 2018/035511

(56) References cited:
- WO-A1-2016/038356
- WO-A1-2017/075096
- WO-A1-2017/075096
- WO-A1-98/41189
- DE-B4- 102011 011 889
- US-A1- 2004 241 302
- US-A1- 2008 020 049
- US-A1- 2009 098 192
- US-A1- 2010 303 883

## Description

### CROSS-REFERENCE TO RELATED APPLICATIONS

This application claims priority to and the benefit of the International Application Nos. PCT/US2017/047702 and PCT/US2017/047703 filed on August 19, 2017, and the U.S. Provisional Application Nos. U.S. 62/377,068 filed on August 19, 2016, U.S. 62/446,431 filed on January 14, 2017, and U.S. 62/468,888 filed on March 8, 2017.

### FIELD OF THE INVENTION

This invention relates generally to methods and apparatuses for the manufacture of fibrous solids, and more particularly to methods and apparatuses for the manufacture of fibrous dosage forms. In some embodiments, the invention herein relates to a method and apparatus for the manufacture of fibrous dosage forms with predictable microstructure.

### BACKGROUND OF THE INVENTION

The most prevalent pharmaceutical dosage forms at present, the porous, oral-delivery tablets, are manufactured by mixing and compacting drug and excipient powders. Although powder processing is extensively used in the manufacture of oral dosage forms, it has inherent limitations: mixing is fraught with particle segregation and agglomeration, and compacting with non-deterministic porosity. As a result, the dosage form microstructure, drug content, and drug release rate are difficult to control tightly, and their range is also limited. Moreover, dosage form manufacture is by a resource-intensive and time-consuming batch process.

Further details about the design and manufacture of state-of-the-art dosage forms are given in the commonly owned references "Remington's Pharmaceutical Sciences XVIII", A.R. Gennaro (ed.), Mack Publishing, Easton, PA, 1990; M.E. Aulton, K.M.G. Taylor, "Aulton's pharmaceutics: The design and manufacture of medicines", fourth edition, Churchill Livingstone, London, UK, 2013; F.J. Muzzio, T. Shinbrot, B.J. Glasser, "Powder technology in the pharmaceutical industry: the need to catch up fast", Powder Technol. 124 (2002) 1-7; T.A. Bell, "Challenges in the scale-up of particulate processes - an industrial perspective", Powder Technol. 150 (2005) 60-71; and "Pharmaceutical Manufacturing Handbook: Regulations and Quality", S. Cox Gad (Ed.), John Wiley & Sons, Inc., Hoboken, NJ, 2008.

The physical difficulties associated with processing granular matter could be overcome, however, by transitioning to the more predictable liquid-based processing. Thus, in the commonly owned U.S. patent application Ser. No. 14/907,891, the U.S. patent application Ser. No. 15/482,776, and the publications in J. Control. Release, 220 (2015) 397-405; Eur. J. Pharm. Biopharm, 103 (2016) 210-218; Int. J. Pharm. 509 (2016) 444-453; Chem. Eng. J. 320 (2017) 549-560; Mater. Sci. Eng. C 80 (2017) 715-727; and Mater. Sci. Eng. C (2017) in press, the present inventors (Blaesi and Saka) have introduced cellular and fibrous dosage forms. Such dosage forms comprise solid frameworks of a drug-excipient solid solution or composite and gas-filled voids or cells. It was shown that both the microstructure and the drug release rate are predictable and precisely controllable; the release rate was predominantly determined by the physico-chemical properties of the excipient, the connectivity of the void space, the cell size (or inter-fiber spacing in the case of fibrous dosage forms), and the wall thickness (or fiber radius).

A method of manufacturing polymeric scaffolds has, for example, been disclosed in the U.S. Patent Application No. US2008/0020049 A1. The scaffold is produced by extruding a scaffolding material from a movable extruder nozzle into a liquid coolant. The coolant has a thermal conductivity greater than 0.026 W/mK to speed up solidification of the extrudate. The disclosed process, however, is not optimal for producing pharmaceutical solid dosage forms. For example, the large mass of the extruder and extruder nozzles compromises their acceleration, and limits the speed and controllability of the nozzle movement. Moreover, the commonly used liquid coolants, the aqueous solutions, are inapplicable for cooling pharmaceutical materials, because pharmaceutical materials generally dissolve in them.

To mitigate such limitations, therefore, in the International Publication No. WO2017/075096 the present inventors (Blaesi and Saka) have introduced a method of manufacturing three-dimensional skeletal structures by depositing plasticized structural elements (e.g., fibers, sheets, etc.) on a movable stage. However, to extrude, deposit, and bond plasticized elements, the elements should be quite fluidic. Fluidic elements deform after deposition, and if the deposited elements are not solidified fast enough, or under appropriate conditions, the patterned microstructure is lost or distorted. The prior art lacks adequate concepts to accelerate or control the solidification rate of the deposited elements; thus the deposited structures are susceptible to excessive or undesirable deformation.

The present invention is defined by the apparatuses according to claims 1, 3 and and the methods according to claims 2, 4 and 6 for the manufacture of pharmaceutical solid dosage forms. The apparatuses and methods according to the invention enable faster and more economical design, development, and manufacture of pharmaceutical dosage forms with repeatable, high quality.

### SUMMARY OF THE INVENTION

In one aspect of the continuous (or semi-continuous or batch) process disclosed herein, one or more solid constituents are first injected into an extrusion channel having a cross section extending along its length inside a housing. Also, at least one solvent is injected into said extrusion channel to solvate at least one injected solid constituent so that the one or more injected solid constituents form a plasticized matrix. The plasticized matrix is then conveyed towards an exit port of the extrusion channel by applying mechanical work on the plasticized matrix. Then the plasticized matrix is extruded through an exit port to form at least one plasticized fiber. Subsequently, said at least one plasticized fiber is structured and solidified to a three dimensional structural network of one or more drug-containing fibers.

In another aspect, one or more solid constituents are first injected into an extrusion channel having a cross section extending along its length inside a housing, wherein at least one solid constituent melts upon heating. The injected one or more solid constituents are then heated to form a plasticized matrix. Concurrently as it is formed or subsequently, the plasticized matrix is conveyed towards an exit port of the extrusion channel by applying mechanical work on the plasticized matrix. The plasticized matrix is then extruded through an exit port to form at least one plasticized fiber. Subsequently, said at least one plasticized fiber is structured and solidified to a three dimensional structural network of one or more drug-containing fibers.

In a further aspect, at least one plasticized matrix is first injected into an extrusion channel having a cross section extending along its length inside a housing. The at least one plasticized matrix is then conveyed towards an exit port of the extrusion channel by applying mechanical work on the at least one plasticized matrix. The at least one plasticized matrix is then extruded through an exit port to form at least one plasticized fiber. Subsequently, said at least one plasticized fiber is structured and solidified to a three dimensional structural network of one or more drug-containing fibers.

The disclosed apparatuses for the manufacture of fibrous dosage forms comprise an internally hollow housing having an internal surface encapsulating and defining an extrusion channel having a first end and a second end and a cross section extending axially along its length from said first end to said second end and terminating into an exit port at the second end.

In one aspect, said housing has at least a first feeding port between the first end and second end for injecting at least one solid constituent into the extrusion channel, and at least a second feeding port between the first feeding port and the exit port for injecting at least one solvent into the extrusion channel to form a plasticized matrix by solvating at least one injected solid constituent. The apparatus further has at least one conveying element for extruding the plasticized matrix in the extrusion channel through an exit port to form at least one plasticized fiber. The apparatus further has a fiber structuring unit to structure and solidify said at least one plasticized fiber to a three dimensional structural network of one or more drug-containing fibers.

In another aspect, said housing has at least a first feeding port between the first end and second end for injecting at least one solid constituent into the extrusion channel. The apparatus further has at least one heating element for fluidizing at least one injected solid constituent so that the injected one or more solid constituents form a plasticized matrix in the extrusion channel. The apparatus further has at least one conveying element for extruding the plasticized matrix in the extrusion channel through an exit port to form at least one plasticized fiber. The apparatus further has a fiber structuring unit to structure and solidify said at least one plasticized fiber to a three dimensional structural network of one or more drug-containing fibers.

In a further aspect, said housing has at least a first feeding port between the first end and second end for injecting at least a plasticized matrix into the extrusion channel. The apparatus further has at least one conveying element for extruding a plasticized matrix in the extrusion channel through an exit port to form at least one plasticized fiber. The apparatus further has a fiber structuring unit to structure and solidify said at least one plasticized fiber to a three dimensional structural network of one or more drug-containing fibers.

In some embodiments, the extrusion channel cross section tapers down before an exit port to the cross section of said exit port.

In some embodiments, the extrusion channel bifurcates into at least one other end comprising an exit port.

According to the claimed invention the apparatuses herein comprise a movable stage for structuring one or more of the extruded plasticized fibers to a three dimensional structural network by depositing said fibers along a path defined by motion of said stage, wherein the stage comprises a grid defining a deposition surface.

According to the claimed invention, the methods herein comprise structuring one or more plasticized fibers to a three-dimensional structural network by depositing said fibers along a path defined by motion of a movable stage.

In some embodiments, the stage comprises an *x-y-z* stage.

According to the claimed invention, the method herein comprises solidifying the deposited three dimensional structural network of one or more fibers by application of a gas flow through said network.

In some embodiments, the stage comprises a grid through which gas flows for solidifying the deposited structure.

In some embodiments, gas flow through the stage promotes solidification of the deposited three dimensional structural network of one or more fibers by evaporating solvent or by cooling.

In some embodiments, the apparatus or method herein further comprise a unit for blowing gas on the deposited structure.

In some embodiments, an apparatus herein further comprises a granular solids feeding unit for injecting one or more granular solid constituents through the first feeding port into the extrusion channel.

In some embodiments, at least one of the one or more injected solid constituents is a granular solid constituent, and wherein said granular solid constituent is injected into the extrusion channel using a granular solids feeding unit.

In some embodiments, an apparatus herein further comprises at least one solvent feeding unit attached to at least the second feeding port for injecting at least one solvent into the extrusion channel.

In some embodiments, injecting at least one solvent into the extrusion channel is performed using a solvent feeding unit.

In some embodiments, at least one conveying element is a screw.

In some embodiments, at least one conveying element is a piston.

In some embodiments, the three dimensional structural network of one or more fibers comprises fiber segments separated and spaced from adjoining fiber segments by free spacings.

In some embodiments, the average thickness of a fiber and an inter-fiber spacing in the three dimensional structural network of fibers are precisely controlled.

In some embodiments, the shear viscosity of a plasticized matrix at an exit port of the extrusion channel is greater than 5 Pa·s.

In some embodiments, the viscosity of at least one plasticized fiber is so that (a) said plasticized fiber bonds to a fiber or a fiber segment on which it is deposited during structuring to a three dimensional structural network of fibers and (b) the 3D-micro-patterned structure is preserved.

In some embodiments, the solid, three dimensiononal structural network of fibers comprises an ordered or partially ordered structure.

In some embodiments, the solid, three dimensional structural network of fibers comprises at least one inter-fiber point contact.

In some embodiments, the solid, three dimensional structural network of fibers comprises at least one inter-fiber point contact, said inter-fiber point contact having a contact width no greater than 2 mm.

In some embodiments, the number of inter-fiber point contacts in the three dimensional structural network of fibers is precisely controlled.

In some embodiments, the position of at least one inter-fiber point contact in the three dimensional structural network of fibers is precisely controlled.

In some embodiments, the position of an inter-fiber contact and the contact width of said inter-fiber contact in the solid, three dimensional structural network of fibers are precisely controlled.

In some embodiments, the one or more drug-containing fibers in the three-dimensional structural network of fibers comprise at least one active ingredient and at least one excipient; said fibers further comprising fiber segments separated and spaced from adjoining fiber segments by free spacings; and the free spacings defining one or more gas-filled free spaces in a drug-containing solid; whereby the one or more fibers in the fibrous dosage form comprise an average thickness no greater than 2.5 mm; the effective free spacing between the fiber segments across the one or more free spaces of the fibrous dosage form on average is greater than 0.1 µm.

In some embodimnents, the one or more drug-containing fibers in the three-dimensional structural network of fibers comprise at least one active ingredient and at least one excipient; said fibers further comprising fiber segments separated and spaced from adjoining fiber segments by free spacings; and the free spacings defining one or more gas-filled free spaces in a drug-containing solid; whereby the one or more fibers in the fibrous dosage form comprise an average thickness between 1 µm and 2.5 mm; the effective free spacing between the fiber segments across the one or more free spaces on average is greater than 1 µm; and the three dimensional structural network of fibers extends over a length, width, and thickness greater than 200 µm.

Additional elements of the method and apparatus disclosed herein are described throughout this specification. Elements of embodiments described with respect to one aspect of the invention can be applied with respect to another aspect without departing from the scope defined by the appended claims. By way of example but not by way of limitation, certain embodiments of the method claims can include features of the apparatus claims, and vice versa.

This invention may be better understood by reference to the accompanying drawings, attention being called to the fact that the drawings are primarily for illustration, and should not be regarded as limiting.

### BRIEF DESCRIPTION OF THE DRAWINGS

The objects, embodiments, features, and advantages of the present invention are more fully understood when considered in conjunction with the following accompanying drawings:
FIG. 1 presents schematic diagrams of the microstructural topology of fibrous dosage forms;
FIG. 2 shows additional schematic diagrams of microstructures of fibrous dosage forms;
FIG. 3 is a schematic of a method and an apparatus for manufacturing fibrous dosage forms;
FIG. 4 is another schematic of a method and an apparatus for manufacturing fibrous dosage forms;
FIG. 5 is a further schematic of a method and an apparatus for manufacturing fibrous dosage forms;
FIG. 6 presents a method and apparatus for manufacturing fibrous dosage forms;
FIG. 7 illustrates a section of a rotating screw or conveying element inside a stationary, hollow housing;
FIG. 8 is a schematic of a tapered fraction of an extrusion channel and an exit port;
FIG. 9 illustrates a micro-patterned microstructure of a fibrous dosage form: (*a*) top view of the fibers in the plane *z* = *z*₀, *(b)* top view of the fibers in the plane *z* = *z*₀ ± 2*R_{f},* and (*c*) isometric view of the microstructure.
FIG. 10 is a schematic illustrating deformation of fibers during or after micropatterning: (*a*) front view of deformation at the contact of crossed fibers, (*b*) top view, and (*c*) front view of a fiber bending downwards;
FIG. 11 schematically illustrates drying of single fiber in convection: (*a*) streamlines of gas flow around the fiber if *Re_{f}* ≈ 60, (*b*) solvent concentration profiles at different times in a fiber cross section if solvent desorption is limited by convection from the fiber surface into the surrounding gas, and (*c*) solvent concentration profiles at different times in a fiber cross section if solvent desorption is limited by solvent diffusion in the fiber;
FIG. 12 presents schematics of streamlines of gas flow around the fibers of a fibrous dosage form in viscous or creeping flow;
FIG. 14 shows schematics of structuring multiple fibers of multiple compositions to a fibrous dosage form structure;
FIG. 15 presents a further schematic of an apparatus and method for manufacturing fibrous dosage forms;
FIG. 16 is a photograph of the apparatus to manufacture the non-limiting experimental examples of melt-processed fibrous dosage forms disclosed herein. The apparatus consists of the components: (a) motor, (b) syringe pump, (c) hopper, (d) extrusion screw and barrel (e.g., housing, extrusion channel, and conveying element), (e) heater, (f) infrared lamp (e.g., heater), (g) extruder nozzle exit (e.g., exit port), (h) deposited fiber bed, (i) supply channels for cooling fluid, and (j) *x-y-z* stage;
FIG. 17 presents photographs of the manufacture of fibrous dosage forms: (*a*) single fiber exiting the extrusion channel, and (b) manufacture of a fibrous dosage form by patterning the fibrous extrudate along the desired path.
FIG. 18 presents data of the rate at which solvent is removed from wet fibers;
FIG. 19 shows scanning electron microscopy (SEM) images of the microstructures of melt-processed single fibers;
FIG. 20 depicts scanning electron microscopy (SEM) images of the microstructures of melt-processed dosage forms;
FIG. 21 depicts scanning electron microscopy (SEM) images of the microstructures of wet-processed single fibers and dosage forms;
FIG. 22 presents the results of the fraction of drug dissolved versus time of melt-processed single fibers;
FIG. 23 displays the results of the fraction of drug dissolved versus time of melt-processed dosage forms;
FIG. 24 shows the results of the fraction of drug dissolved versus time of wet-processed single fibers and dosage forms;
FIG. 25 presents the shear viscosity of a water-excipient-drug suspension versus shear strain rate. The composition of the suspension is: 40 wt% water, 36 wt% drug, and 24 wt% excipient;
FIG. 26 shows the results of shear viscosity measurements of additional water-excipient solutions versus weight fraction of the polymeric excipient. Polyvinyl alcohol-polyethylene glycol graft copolymer 3:1 with a molecular weight of 45,000 Daltons (tradename: Kollicoat IR) was the excipient in this case.

### DEFINITIONS

In order for the present disclosure to be more readily understood, certain terms are first defined below. Additional definitions for the following terms and other terms are set forth throughout the specification.

In this application, the use of "or" means "and/or" unless stated otherwise. As used in this application, the term "comprise" and variations of the term, such as "comprising" and "comprises" are not intended to exclude other additives, components, integers or steps. As used in this application, the terms "about" and "approximately" are used as equivalents. Any numerals used in this application with or without about/approximately are meant to cover any normal fluctuations appreciated by one of ordinary skill in the relevant art.

Moreover, in the disclosure herein, the terms "one or more active ingredients", "active ingredient", "active pharmaceutical ingredient", and "drug" are used interchangeably. As used herein, an "active ingredient" or "active agent" refers to an agent whose presence or level correlates with elevated level or activity of a target, as compared with that observed absent the agent (or with the agent at a different level). In some embodiments, an active ingredient is one whose presence or level correlates with a target level or activity that is comparable to or greater than a particular reference level or activity (e.g., that observed under appropriate reference conditions, such as presence of a known active agent, e.g., a positive control).

A granular solid in this disclosure is a conglomeration of discrete solid particles of a specific composition. The average size of the particulates (e.g., the average diameter or the third root of the average volume) may be in the range from about 0.001 µm or smaller to about 150 mm or greater. This includes, but is not limited to an average size of the particulates of 0.002 µm - 200 mm, 0.005 µm - 200 mm, 0.01 µm - 150 mm, 0.05 µm - 150 mm, 0.1 µm - 150 mm; 1 µm - 100 mm, 1 µm - 50 mm, 5 µm - 50 mm, 10 µm - 50 mm, or 5 µm - 30 mm.

In the context of the invention herein, a plasticized matrix is a viscous material comprising a minimum shear viscosity of 0.005-5×10⁹ Pa·s at a shear rate no greater than 10 1/s. This includes but is not limited to a minimum shear viscosity of 0.01-5×10⁹ Pa·s, 0.025-10,000,000 Pa·s, 0.05-5,000,000 Pa·s, 0.1-2,000,000 Pa·s, 0.25-1,000,000 Pa·s, 0.25-5,000,000 Pa·s, 0.5-2,000,000 Pa·s, 1-2,000,000 Pa·s, 1-5,000,000 Pa·s, 1-1,000,000 Pa·s, or 1-500,000 Pa·s at a shear rate no greater than 10 1/s. Non-limiting examples of a plasticized matrix include but are not limited to polymer melts, concentrated solutions of one or more polymers and one or more solvents (e.g., water, ethanol, acetone, isopropanol, etc.), suspensions of solid particulates or granules and a polymer melt, or suspensions of solid particulates and a concentrated polymeric solution, etc. It may be noted that in the context of the invention herein the terms "plasticized matrix", "plasticized material", and "melt" are used interchangeably.

Furthermore, in the context of the invention herein, a three dimensional structural network of drug-containing fibers comprises a drug-containing fibrous structure (e.g., an assembly or an assemblage of one or more fibers) that extends over a length, width, and thickness greater than 200 µm. This includes, but is not limited to drug-containing fibrous structures that extend over a length, width, and thickness greater than 300 µm, or greater than 500 µm, or greater than 700 µm, or greater than 1 mm, or greater than 1.25 mm, or greater than 1.5 mm, or greater than 2 mm.

As used herein, the terms "fiber", "fibers", "one or more fibers", "one or more drug-containing fibers", and "drug-containing fibers", are used interchangeably. They are understood as the solid, drug-containing structural elements (or building blocks) that make up the three dimensional structural network of drug-containing fibers (e.g., the structure of a fibrous dosage form). A fiber has a length much greater than its width and thickness (e.g., the length of a fiber is much greater than its width and the length of a fiber is much greater than its thickness). In the present disclosure, a fiber is referred to as having a length greater than 2 times its width and thickness. This includes, but is not limited to a fiber length greater than 3 times, or greater than 4 times, or greater than 5 times, or greater than 6 times, or greater than 8 times, or greater than 10 times, or greater than 12 times the fiber width and thickness. In yet other embodiments that are included but not limiting in the disclosure herein, the length of a fiber may be greater than 0.3 mm, or greater than 0.5 mm, or greater than 1 mm, or greater than 2.5 mm.

Moreover, as used herein, the term "fiber segment" refers to a fraction of a fiber along the length of said fiber.

In the invention disclosed herein, fibers (or fiber segments) may be bonded, and thus they may serve as building blocks of "assembled structural elements" with a geometry different from that of the original fibers. Such assembled structural elements include two-dimensional elements (or 2-dimensional structural elements), one-dimensional elements (or 1-dimensional structural elements), or zero-dimensional elements (or 0-dimensional structural elements).

As used herein, a two-dimensional structural element is referred to as having a length and width much greater than its thickness. In the present disclosure, the length and width of a two-dimensional sructural element are greater than 2 times its thickness. An example of such an element is a "sheet". A one-dimensional structural element is referred to as having a length much greater than its width and thickness. In the present disclosure, the length of a one-dimensional structural element is greater than 2 times its width and thickness. An example of such an element is a "fiber". A zero-dimensional structural element is referred to as having a length and width of the order of its thickness. In the present disclosure, the length and width of a zero-dimensional structural element are no greater than 2 times its thickness. Furthermore, the thickness of a zero-dimensional element is less than 2.5 mm. Examples of such zero-dimensional elements are "particles" or "beads" and include polyhedra, spheroids, ellipsoids, or clusters thereof.

### DETAILED DESCRIPTION OF THE INVENTION

### Fibrous dosage forms

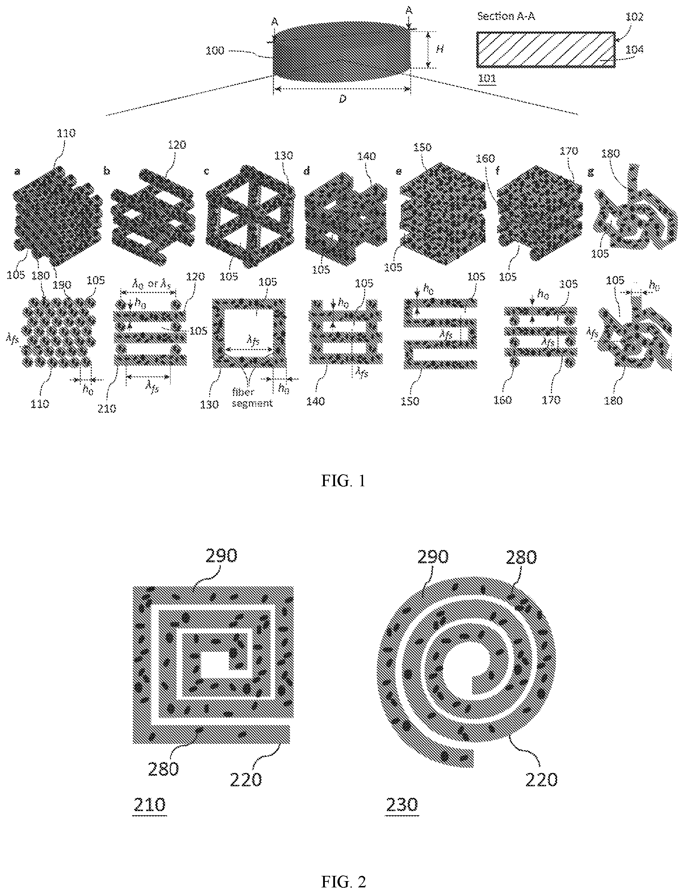

In the context of the invention herein, a fibrous dosage form is referred to a dosage form that is prepared from at least one fiber. This includes, but is not limited to dosage forms that are prepared from at least one building block of one or more fibers (e.g., at least one fibrous building block). Thus, as shown in the non-limiting schematics of FIG. **1****,** a fibrous dosage form **100** may comprise a drug-containing solid **101** having an outer surface **102** and an internal structure **104** contiguous with and terminating at said outer surface **102.** The internal structure **104** may comprise a three dimensional structural network of one or more drug-containing fibers **110, 120, 130, 140, 150, 160, 170, 180.** The fibers may further comprise fiber segments separated and spaced from adjoining fiber segments by free spacings, *λ_{fs}*, which define one or more free spaces **105** in the drug-containing solid **101.**

The fibers **110, 120, 130, 140, 150, 160, 170, 180** may be oriented (e.g., arranged or structured) in a variety of ways, ranging from random (e.g., disordered as shown in FIG. **1g**) to partially regular (e.g., partially ordered) to regular (e.g., ordered or not random as shown in FIGS. **1a-f**). In a regular structure, the position and thickness of the one or more fibers are typically precisely controlled. Dosage forms comprising such precisely controlled structures enable precisely controlled, predictable and consistent properties. This is desirable for optimizing the dosage form towards a specific functionality, such as the drug release rate (or the concentration-time profile of drug in the blood plasma after ingestion of the dosage form), the dosage form's mechanical properties, etc.

Furthermore, as shown in FIG. 1, in some embodiments the three dimensional structural network of one or more fibers may comprise inter-fiber contacts (e.g., contacts between fibers and/or fiber segments) which by way of example but not by way of limitation can be point contacts (as schematically shown in FIGS **1b** and **1d**) or line contacts as schematized in FIGS **1a**, **1e** and **1f** (for further information related to point contacts and line contacts, *see, e.g.,* K.L. Johnson, "Contact mechanics", Cambridge University Press, 1985). Generally, a point contact is referred to as having a contact area (or a contact width) between two contacting fibers (or fiber segments) smaller than the dimension (e.g., the projected area or the width or the length, etc.) of the dosage form (or the drug-containing solid or the three dimensional structural network of fibers).

Such inter-fiber contacts may provide mechanical support to the fibrous structure (e.g., the three dimensional structural network of one or more fibers). They may, however, also hold up disintegration and dissolution of the fibrous structure upon immersion in a dissolution medium. Thus, in some embodiments the number of inter-fiber contacts, and/or at least one position of an inter-fiber contact, and/or a contact width of at least one inter-fiber contact are precisely controlled in the three dimensional structural network of one or more fibers.

Moreover, as shown in the non-limiting examples of three dimensional structural networks of fibers shown in FIGS. **1e** and **1f**, the fibers may be bonded to each other along a line contact to form one or more 2-dimensional structural elements (e.g., one or more sheets). In FIG. **1e**, the fibrous structure comprises one such 2-dimensional structural element that is continuous (e.g., one continuous sheet) **150.** In FIG. **1f** the fibrous structure comprises a combination of fibers **160** and sheets **170.**

Other non-limiting examples of three dimensional structural networks of fibers are shown in FIG. **2****,** which presents a top view of fibers **220** in a plane forming a rectangular structure **210,** as well as a top view of fibers **220** in a plane forming a circular (or elliptical) structure **230.**

Additional non-limiting examples of fibrous structures can be found in the commonly owned references M.F. Ashby, "The mechanical properties of cellular solids", Metall. Trans. A, 14A (1983) 1755-1769; L.J. Gibson, M.F. Ashby, "Cellular solids: structure and properties", second edition, Cambridge University Press, 1999; L.J. Gibson, M.F. Ashby, "Cellular solids in medicine", second edition, Cambridge University Press, 1999. Moreover, further details related to fibrous dosage forms can be found in the U.S. patent application Ser. No. 15/482,776.

Any more examples of fibrous dosage forms or fibrous structures would be obvious to a person of ordinary skill in the art.

### Compositions

Typically, the compositions of the one or more solid or semi-solid fibers comprising the building blocks (or at least one building block) of a fibrous dosage form include at least one active ingredient. By way of example but not by way of limitation, said active ingredient may be selected from the group comprising acetaminophen, aspirin, caffeine, ibuprofen, an analgesic, an anti-inflammatory agent, an anthelmintic, anti-arrhythmic, antibiotic, anticoagulant, antidepressant, antidiabetic, antiepileptic, antihistamine, antihypertensive, antimuscarinic, antimycobacterial, antineoplastic, immunosuppressant, antihyroid, antiviral, anxiolytic and sedatives, beta-adrenoceptor blocking agents, cardiac inotropic agent, corticosteroid, cough suppressant, diuretic, dopaminergic, immunological agent, lipid regulating agent, muscle relaxant, parasympathomimetic, parathyroid, calcitonin and biphosphonates, prostaglandin, radiopharmaceutical, anti-allergic agent, sympathomimetic, thyroid agent, PDE IV inhibitor, CSBP/RK/p38 inhibitor, or a vasodilator, among others.

In some embodiments, the one or more solid or semi-solid fibers comprising the building blocks (or at least one building block) of a fibrous dosage form further comprise at least one pharmaceutical excipient. Non-limiting examples of excipients include polyethylene glycol (PEG), polyethylene oxide, polyvinylpyrrolidone (PVP), PEG-PVP copolymer, poloxamer, lauroyl macrogol-32 glycerides, polyvinylalcohol (PVA), PEG-PVA copolymer, polylactic acid, polyvinylacetate phthalate, polymethacrylates (e.g., poly(methacrylic acid, ethyl acrylate) 1:1, or butylmethacrylat-(2-dimethylaminoethyl)methacrylat-methylmathacrylat-copolymer), gelatin, cellulose or cellulose derivatives (e.g., microcrystalline cellulose, hydroxypropyl cellulose, hydroxyethyl cellulose, methyl cellulose, hydroxypropyl methyl ether cellulose, hydroxypropyl methylcellulose, hydroxypropyl methylcellulose acetate succinate, hydroxypropyl methylcellulose phthalate, cellulose acetate phthalate, etc.), starch, polylactide-co-glycolide, polyvinyl caprolactam-polyvinyl acetate-polyethylene glycol graft copolymer, lactose, starch derivatives (e.g., pregelatinized starch or sodium starch glycolate, etc.), chitosan, pectin, polyols (e.g., lactitol, maltitol, mannitol, isomalt, etc.), acrylic acid crosslinked with allyl sucrose or allyl pentaerythritol (e.g., carbopol), sodium alginate, or polyacrylic acid, among others.

The one or more free spaces may be filled with a gas, or a liquid, or nothing (e.g., a vacuum). Gases that may fill the one or more free spaces include, but are not limited to one of air, oxygen, argon, nitrogen, CO₂, nitric oxide, or helium, among others. Similarly, liquids to fill the one or more free spaces may be selected from the group comprising water, olive oil, Polyethylene glycol (PEG) with molecular weight smaller than about 1000 Da (e.g. PEG 400, PEG 300, etc.), Poloxamer 124, 2-Pyrrolidone, Glycerol triacetate (Triacetin), D-alpha tocopheryl polyethylene glycol 1000 succinate (TPGS), Polyoxyl Hydroxystearate, Polyoxyl 15 Hydroxystearate, Castor oil, Polyoxyl castor oil (Polyethoxylated castor oil), Polyoxyl 35 castor oil, Polyoxyl hydrogenated castor oil, Glyceryl monooeleate, Glycerin, Propylene glycol, Propylene carbonate, Propionic acid, Peanut oil, water, Sesame oil, Almond oil, combinations of such (and/or other) liquids with a polymer or any other molecule that dissolves in them, among others.etc.

Any more examples of the compositions of the one or more fibers and free spaces of the fibrous dosage forms would be obvious to a person of ordinary skill in the art.

### Solid structures of fibers

The solid structure of a fiber may comprise a single-phase or multiple phases. If a fiber consists of multiple phases, such as at least a drug phase and at least an excipient phase, the phases (e.g., the drug and excipient phases) may be structured in the fiber in an ordered or partially or completely disordered manner. The structural features of the individual phases may comprise particles, beads, polygons, ellipsoids, cubes, tubes, rods, any combinations thereof, etc. The size (e.g., the length or diameter) of the structural features may be at the, molecular, nano-, micro-, meso-, or macro-scale. Thus, by way of example but not by way of limitation, solid structures of fibers comprising drug molecules that are dissolved or embedded in an excipient (e.g., one or more excipients) are included in the solid structures of fibers of this disclosure. Also, solid structures of fibers comprising drug particles that are embedded in an excipient are included herein.

More such examples of solid structures of fibers would be obvious to a person of ordinary skill in the art.

### Aspects of the method

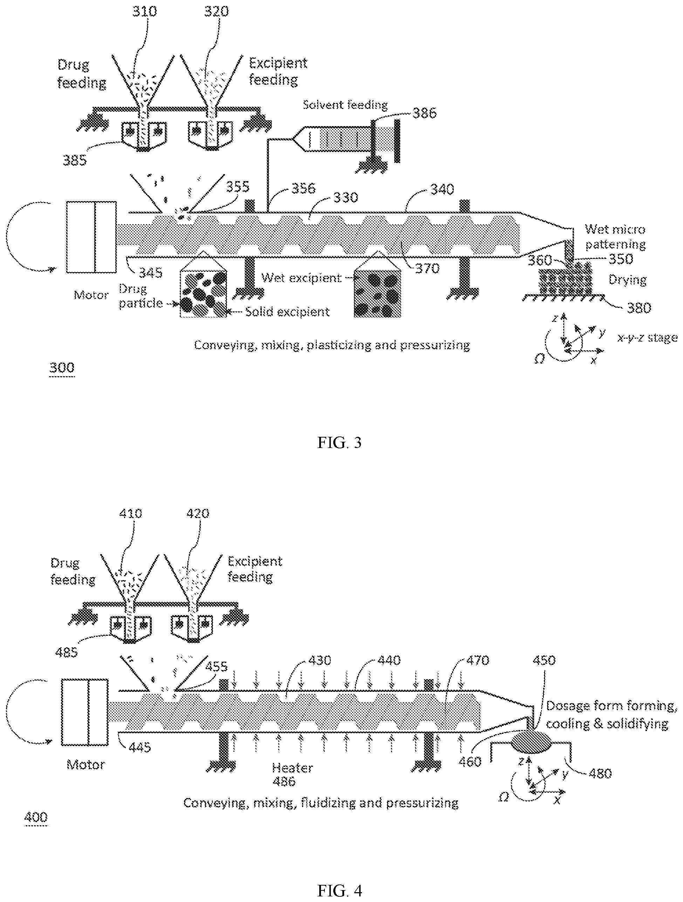

FIG. 3 presents a non-limiting example of a method of manufacturing pharmaceutical solid dosage forms according to this invention. One or more drugs 310 and/or one or more solid excipients 320 (the one or more drugs 310 and/or one or more solid excipients 320 combined are also referred to herein as "one or more solid constituents") are fed or injected into an extrusion channel **330** having a cross section extending along its length inside a housing **340.** Also, at least one solvent that solvates at least one injected solid constituent is injected into the extrusion channel **330.** The amount of solvent injected and the volume fraction of the at least one solvated solid constituent with respect to the total volume of the one or more injected solid constituents are so that the one or more injected solid constituents form a plasticized matrix upon contact and mixing with the solvent. The plasticized matrix is (subsequently or concurrently as it is formed) conveyed towards an exit port **350** of the extrusion channel **330** by applying mechanical work on the plasticized matrix (e.g., by applying a shear force on the plasticized matrix along a fraction of the extrusion channel **430,** or by applying a pressure gradient in the direction of the extrusion channel **330,** etc.). The plasticized matrix is then extruded through an exit port **350** to form at least one plasticized fiber **360.** Subsequently, said at least one plasticized fiber **360** (e.g., one or more plasticized fibers) is/are structured and solidified to a three dimensional structural network of one or more drug-containing fibers. It may be noted that in the invention herein, the one or more drug-containing fibers in the three-dimensional structural network of fibers typically comprise at least one active ingredient and at least one excipient; said fibers typically further comprise fiber segments separated and spaced from adjoining fiber segments by free spacings; and the free spacings typically define one or more gas-filled free spaces in a drug-containing solid (e.g., in a solid dosage form, or in a fibrous dosage form, etc.).

Another non-limiting example of a method of manufacturing pharmaceutical solid dosage forms according to this invention is shown in FIG **4****.** One or more drugs **410** and/or one or more solid excipients **420** (e.g., one or more solid constituents) are fed or injected into an extrusion channel **430** having a cross section extending along its length inside a housing **440.** The injected one or more solid constituents are then heated to a temperature greater than the melting temperature of at least one injected solid constituent. Thus at least one injected solid constituent is fluidized (e.g., it transitions from solid or solid-like to fluidic or fluid-like) upon heating. The volume fraction of the fluidized solid constituent (or the fluidized solid constituents) with respect to the volume of the one or more injected solid constituents is so that the one or more injected solid constituents form a plasticized matrix upon heating (and mixing). The plasticized matrix is (subsequently or concurrently as it is formed) conveyed towards an exit port **450** of the extrusion channel **430** by applying mechanical work on the plasticized matrix (e.g., by applying a shear force on the plasticized matrix along a fraction of the extrusion channel **430,** or by applying a pressure gradient in the direction of the extrusion channel **430,** etc.). The plasticized matrix is then extruded through an exit port **450** to form at least one plasticized fiber **460.** Subsequently, said at least one plasticized fiber **460** (e.g., one or more plasticized fibers) is/are structured and solidified to a three dimensional structural network of one or more drug-containing fibers.

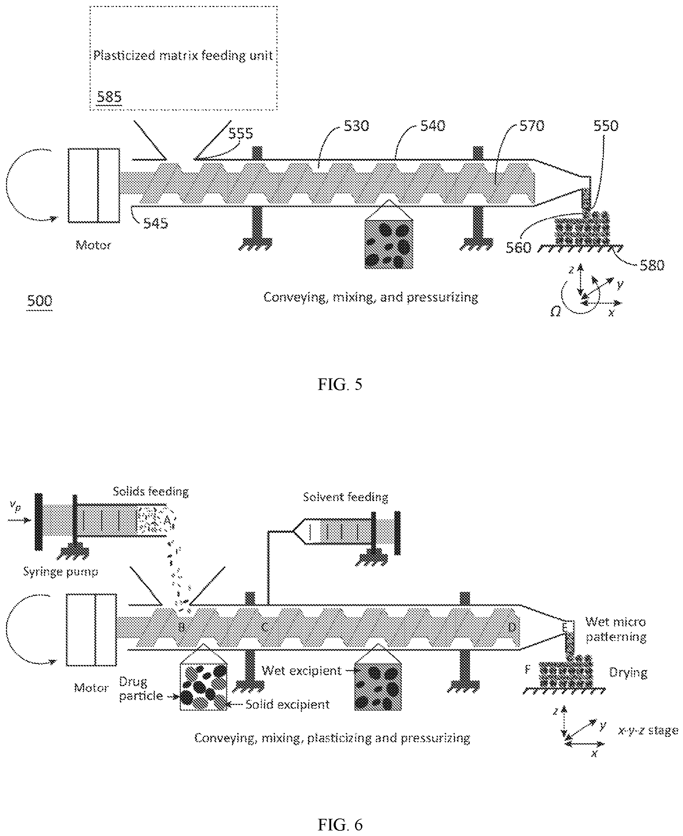

FIG. **5** presents another non-limiting example of a method of manufacturing pharmaceutical solid dosage forms according to the invention herein. At least one plasticized matrix is fed or injected into an extrusion channel **530** having a cross section extending along its length inside a housing **540.** The plasticized matrix in the extrusion channel is then conveyed towards an exit port **550** of the extrusion channel **530** by applying mechanical work on the plasticized matrix (e.g., by applying a shear force on the plasticized matrix along a fraction of the extrusion channel **530,** or by applying a pressure gradient in the direction of the extrusion channel **530,** etc.). The plasticized matrix is then extruded through an exit port **550** to form at least one plasticized fiber **560.** Subsequently, said at least one plasticized fiber **560** (e.g., one or more plasticized fibers) is/are structured and solidified to a three dimensional structural network of one or more drug-containing fibers.

It may be noted that in any example presented herein, the extrusion channel **330, 430, 530** may comprise one or multiple exit ports through which plasticized material can be extruded.

Any more examples of the process steps to manufacture the fibrous dosage forms would be obvious to a person of ordinary skill in the art.

### Aspects of the apparatus

FIG. 3 also presents a non-limiting example of an apparatus **300** for the manufacture of pharmaceutical solid dosage forms according to this invention. The apparatus **300** comprises an internally hollow housing **340** having an internal surface encapsulating and defining an extrusion channel **330** having a first end **345** and a second end **350** and a cross section extending axially along its length from said first end **345** to said second end **350** and terminating into an exit port **350** at the second end **350.** The housing **340** further has at least a first feeding port **355** between the first end **345** and second end **350** for feeding or injecting at least one solid constituent into the extrusion channel **330.** Moreover, the housing **340** has at least a second feeding port **356** between the first feeding port **355** and the exit port **350** for injecting at least one liquid into the extrusion channel **330** to form a plasticized matrix by solvating at least one injected solid constituent. The apparatus **300** further comprises at least one conveying element **370** for extruding the plasticized matrix in the extrusion channel through an exit port **350** to form at least one plasticized fiber **360.** The apparatus **300** further comprises a fiber structuring unit **380** to structure said at least one plasticized fiber **360** (e.g., one or more plasticized fibers) to a three dimensional structural network of one or more drug-containing fibers. In some embodiments, the apparatus **300** further comprises a solid constituent feeding unit **385** (e.g., a granular solid feeding unit) for injecting at least one solid constituent (e.g., at least one granular solid constituent) through the first feeding port **355** into the extrusion channel **330.** Furthermore, in some embodiments the apparatus further comprises a solvent feeding unit **386** attached to the second feeding port **356** for injecting at least one solvent into the extrusion channel **330.**

Another non-limiting schematic of an apparatus **400** for the manufacture of pharmaceutical solid dosage forms according to this invention is shown in FIG. **4****.** The apparatus 400 comprises an internally hollow housing **440** having an internal surface encapsulating and defining an extrusion channel 4**30** having a first end **445** and a second end **450** and a cross section extending axially along its length from said first end **445** to said second end **450** and terminating into an exit port **450** at the second end **450.** The housing **440** further has at least a first feeding port **455** between the first end **445** and second end **450** for feeding or injecting at least one solid constituent into the extrusion channel **430.** The apparatus **400** further comprises at least one heating element **486** for fluidizing at least one injected solid constituent so that the injected one or more solid constituents form a plasticized matrix in the extrusion channel **430.** The apparatus **400** further has at least one conveying element **470** for extruding the plasticized matrix in the extrusion channel **430** through an exit port **450** to form at least one plasticized fiber **460.** The apparatus **400** further has a fiber structuring unit **480** to structure said at least one plasticized fiber **460** (e.g., one or more plasticized fibers) to a three dimensional structural network of one or more drug-containing fibers. In some embodiments, the apparatus **400** further comprises a solid constituent feeding unit **485** (e.g., a granular solid feeding unit) for injecting at least one solid constituent (e.g., at least one granular solid constituent) through the first feeding port **455** into the extrusion channel **430.**

FIG. **5** presents yet another non-limiting example of an apparatus **500** according to the invention herein. The apparatus **500** comprises an internally hollow housing **540** having an internal surface encapsulating and defining an extrusion channel **530** having a first end **545** and a second end **550** and a cross section extending axially along its length from said first end **545** to said second end **550** and terminating into an exit port **550** at the second end **550.** The housing **540** further has at least a first feeding port **555** between the first end **545** and second end **550** for feeding or injecting a plasticized matrix into the extrusion channel **530.** The apparatus **500** further has at least one conveying element **570** for extruding the plasticized matrix in the extrusion channel **530** through an exit port **550** to form at least one plasticized fiber **560.** The apparatus **500** further comprises a fiber structuring unit **580** to structure said at least one plasticized fiber **560** (e.g., one or more plasticized fibers) to a three dimensional structural network of one or more drug-containing fibers. In some embodiments, the apparatus **500** further comprises a plasticized matrix feeding unit **585** for injecting at least one plasticized matrix through the first feeding port **555** into the extrusion channel **530.**

In any example presented herein, the extrusion channel **330, 430, 530** may comprise one or multiple exit ports through which a plasticized fiber may be extruded.

Any more examples of an apparatus to manufacture the fibrous dosage forms would be obvious to a person of ordinary skill in the art.

### Process Models

The following non-limiting examples set forth, in detail, ways by which specific non-limiting examples of the process and apparatus disclosed may be modeled. The models will enable one of skill in the art to more readily understand the invention and its features. The models and examples are presented by way of illustration, and are not meant to be limiting in any way.

### (a) Process overview

The models presented refer mostly to the non-limiting apparatus and method illustrated schematically in FIG. 6. FIG. 6 is a schematic of the apparatus and method applied for preparing the non-limiting experimental examples of wet-processed fibrous dosage forms shown later in this specification.

As shown in FIG. 6, the input material is a mixture of one or more granular solids, which are filled in a syringe with uniform barrel diameter at point A. By controlled displacement of the piston of the syringe pump the powder mixture (e.g., the one or more granular solids) is fed (e.g., injected) through a hopper into an extrusion channel at B. In the extrusion channel, multiple unit steps are integrated into a continuous process. First, the solid granules are conveyed away from the inlet by a conveying element, which in the non-limiting example presented is a rotating screw. Second, the one or more granular solids are mixed further as they are conveyed forward along the screw. Third, because a solvent is added at point C that solvates at least one injected granular solid, said at least one injected granular solid transitions from solid or solid-like to fluidic or fluid-like and plasticizes the mixture (e.g. the one oner more granular solids) as it is transported forward. The mixture is plasticized (e.g., the one or more granular solids are converted to a plasticized matrix) soon after point C. Thus fourth, in section CD the pressure of the plasticized matrix increases as it is conveyed forward along the screw. The pressure generated by the extruder screw drives the fluid stream (e.g., the plasticized matrix) through a converging region of the extrusion channel from D to E, and thereafter through an exit port. Thus fifth, a plasticized fiber is formed as the plasticized matrix is extruded through said exit port of the extrusion channel. The plasticized fiber is subsequently deposited on a stage or substrate that moves with respect to the exit port so that a 3D-micro-patterned fibrous dosage form structure is formed at F. Finally, the wet fibrous structure is dried to form a solid fibrous dosage form.

### (b) Injecting granular solids into the extrusion channel

In the non-limiting example presented in FIG. 6, the rate at which the one or more granular solids are fed or injected into the extrusion channel is controlled volumetrically. In a volumetric, continuous particulate feeder (e.g., a volumetric, continuous granular solid feeding unit), the mass flow rate, *dM_{sd}*/*dt,* of solid particles (e.g., one or more granular solids) fed to the extrusion channel may be written as: $\frac{{dM}_{sd}}{dt} = {ρ}_{p} {ϕ}_{p} {A}_{p} {ν}_{p}$ (1) where *ρₚ* is the density (or average density) of the solid particles, *φₚ* their volume fraction in the particle bed, *Aₚ* the cross-sectional area of the particle bed, and *νₚ* its translational velocity. *νₚ* may be controlled, for example, by the axial velocity of a piston, the rotation rate of a conveyor screw, or other methods.

The mass flow rate of particulates in volumetric feeders is typically subject to some variation because no rigorous mathematical models are available that adequately describe the behavior of granular matter. Consequently, *φₚ* and *νₚ* must typically be found from heuristic models. In some cases they are difficult to control precisely.

More precise control of the mass flow rate of particulates fed to the extrusion channel may be achievable, however, by gravimetric control. In a gravimetric continuous particulate feeder, the mass flow rate of particulates injected is directly measured and controlled (e.g., the weight of the one or more granular solids supplied to the extrusion channel per unit time is continuously measured and controlled). Thus, despite the difficulties in modeling the flow and behavior of granular matter, fairly precise control of the rate at which particulates are fed to the extrusion channel is achievable.

Such precise control can be desirable, for example, if multiple granular solids are injected into the extrusion channel and each granular solid is injected with a different feeder. Precise control of the rate at which each constituent (e.g., each granular solid) is injected ensures that the mass- or volume fractions of the components (e.g., the constituents) in the extrusion channel are precisely controlled (e.g., the mass- or volume fraction of each constituent in the extrusion channel is precisely controlled).

It may be noted that in some embodiments, a granular solid (e.g., one or more particles) may consist of multiple components, such as one or more drugs and/or one or more excipients. Furthermore, it may be noted that the models and concepts presented above are not limiting. By way of example but not by way of limitation, also one or more plasticized matrices may be injected into the extrusion channel by volumetric or gravimetric control of the flow rate. Furthermore, it would be obious to a person of ordinary skill in the art that any material (e.g., one or more granular solids, one or more plasticized matrices, etc.) can also be fed to the extrusion channel in a batch mode or in a semi-continuous mode.

Any more models or examples for injecting granular solids or one or more plasticized matrices or even one or more solid materials such as filaments, sheets, rods, etc. into the extrusion channel would be obvious to a person of ordinary skill in the art.

### (c) Fluidizing at least one granular solid in the extrusion channel

The input to the non-limiting process shown in FIG. 6 is a solid material, thus solids handling is unavoidable at the beginning in this case. But because at least one of the injected granular solids transitions from solid or solid-like to fluidic or fluid-like upon contact with a solvent (e.g., water, acetone, dimethylsulfoxide, ethanol, ethyl acetate, etc.), said at least one granular solid may be fluidized by solvation or dissolution in the solvent. If the flux of the solvent molecules to the interior of a solid particle is faster than the flux of the particle's molecules into the solvent, the time to fluidize said particle by solvation, *tₛₒₗᵥ,* is roughly equal to the time the solvent molecules require to diffuse to the center of said particle. Thus for a solid particle of radius, *Rₚ*, in which diffusion of the solvent is Fickian, ${t}_{solv} ≅ \frac{{R}_{p}^{2}}{{D}_{eff}}$ (2) where *D_{eff}* the effective diffusivity of the solvent in the particle. By way of example but not by way of limitation, if *Rₚ* = 25 µm, *D_{eff}* = 3×10⁻¹⁰ m²/s (e.g., of the order of the diffusion coefficient of water in hydroxypropyl methyl cellulose), *tₛₒₗᵥ =* 2.1 s. Thus for a screw rotating at 5-500 rpm (this range is used here by way of example and not by way of limitation), if each particle is surrounded by the solvent immediately after the solvent is added, the one or more granular solids are converted to a wet, plasticized material (e.g., a plasticized matrix) about 5×2.9/60 - 500×2.9/60 = 0.17-17 screw turns after the solvent feeding position. Thus, a granular solid may be readily fluidized by solvation in an apparatus or method as illustrated in FIG. 6.

Alternatively, it has been reported that in a typical extruder screw and barrel, from the point where the barrel temperature exceeds the melting temperature of a thermoplastic solid, at about 2-4 times the pitch of the screw forward, a small melt pool forms (*see, e.g.,* C.G. Gogos, Z. Tadmor, "Principles of polymer processing", second edition, John Wiley & Sons, 2006). Thus a thermoplastic granular solid can also be readily fluidized by melting in a system as shown, for example, in the non-limiting schematic of FIG. 4.

Any more examples or models to fluidize the one or more injected granular solids in the extrusion channel would be obvious to a person of ordinary skill in the art.

### (d) Applying mechanical work on the plasticized matrix in the extrusion channel

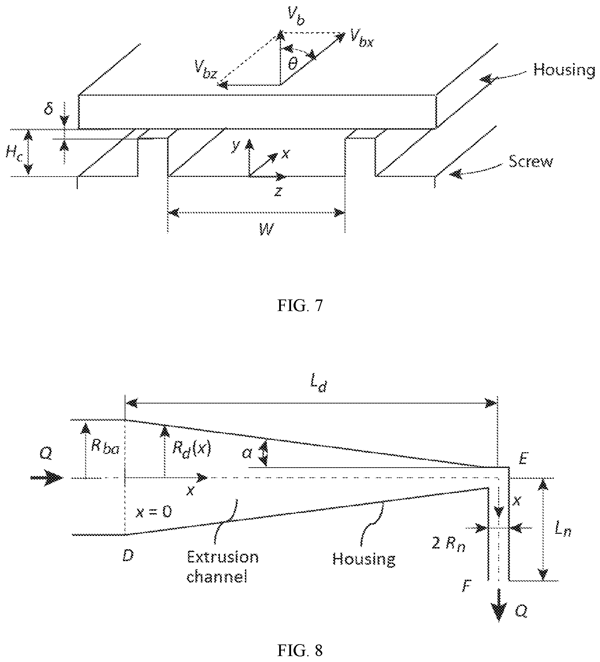

If mechanical work is applied on the plasticized matrix, it flows. In the non-limiting examples of FIGS. **6** and **7****,** mechanical work is applied on the plasticized matrix in the extrusion channel by a rotating extrusion screw. Thus the screw transports the plasticized material towards an exit port of the extrusion channel. The melt flow rate in the *x*-direction (e.g., the throughput of the extruder), *Q*, may be approximated by: $Q = {Q}_{d} + {Q}_{p}$ (3) where *Q_{d}* is the drag- and *Qₚ* the pressure-driven flow contribution. The drag or Couette flow, *Q_{d},* is due to the rotation of the housing relative to the screw (or vice versa) and drives the material forward. It may be approximated as follows: ${Q}_{d} = \frac{{V}_{bx} {WH}_{c}}{2} = \frac{2 {πR}_{ba} {N}_{ba} cos {θWH}_{c}}{2}$ (4) where *V_{bx}* is the velocity of the housing relative to the screw in the *x*-direction, *W* the width of the screw channel, *H_{c}* the height of the screw channel, *R_{ba}* the radius of the extrusion channel (e.g., the internal radius of the internally hollow housing), *N_{ba}* the rotation rate of the housing relative to the screw, and *θ* the helix angle of the screw.

The pressure-driven flow rate, *Qₚ,* drives the material backward. If the plasticized matrix is Newtonian viscous and the flow laminar, ${Q}_{p} = - \frac{{WH}_{c}^{3}}{12 {μ}_{m}} \frac{{P}_{D} - {P}_{C}}{L}$ (5) where *µₘ* is the viscosity of the plasticized matrix and *L* the length of the screw channel in the *x*-direction that is completely filled with the plasticized material.

Thus if, by way of example but not by way of limitation, *R_{ba} =* 5 mm, *θ* = 17°, *W* = 10 mm, and *H_{c}* = 1 mm, the drag flow, *Q_{d} =* 13 - 1252 mm³/s for *N_{ba} =* 5-500 rpm. Under these conditions, if the pressure at C, *P_{C},* is atmospheric, *L* = 25 cm and *µₘ* = 115 Pa·s, the pressure at *D, P_{D},* is between atmospheric and 3.9 MPa. Thus in this non-limiting example, a variety of throughputs (or flow rates) and a variety of fluid pressures can be generated by applying mechanical work on the plasticized matrix in the extrusion channel.

It may be noted that mechanical work may alternatively be applied by the axial displacement of a piston in contact with the plasticized matrix, a peristaltic pump, or pressurized gas or liquid, among others.

Any more models or examples to apply mechanical work on the plasticized matrix in the extrusion channel so as to convey it towards an exit port would be obvious to a person of ordinary skill in the art.

### (e) Fluid flow and pressure drop in the extrusion channel

Under steady-state conditions, the mass- and volumetric flow rates, *dM*/*dt* and *Q,* of plasticized material in section CF of the extrusion channel are determined by the rates at which granular solids and solvent are injected: $\frac{dM}{dt} = \frac{{dM}_{sd}}{dt} + {ρ}_{solv} {Q}_{solv}$ (6a) $Q = \frac{1}{ρ} \times \frac{dM}{dt}$ (6b) where *ρₛₒₗᵥ* is the density and *Qₛₒₗᵥ* the volumetric feeding rate of the solvent, and *ρ* the density of the plasticized material. Under the conditions of the non-limiting experimental example 4 shown later, *dM_{sd}*/*dt =* 1.7 mg/s, *ρₛₒₗᵥ* = 1000 kg/m³, *Qₛₒₗᵥ =* 1.13 mm³/s, and *ρ =* 1000 kg/m³. Thus by Eqs. (6a) and (6b), *Q* = 2.83 mm³/s.

FIG. **8** shows non-limiting examples of a fraction of an extrusion channel that terminates into an exit port and tapers down before said exit port. The average velocity of the plasticized material in said fraction of the extrusion channel, *ν* ≤ *Q*/*πRₙ*², where *Rₙ* is the radius of the extrusion channel at the smallest cross section (which in the non-limiting examples of FIGS. **6** and **8** is the radius of the exit port). Thus the Reynolds number, *Re =* 2*ρνRₕ*/*µₛ* ≤ 2*ρQ*/*πµ_{s,n}Rₙ =* 1.8×10⁻⁴ and the capillary number, *Ca = µₛν*/*γ* ≈ *µ_{s,n}Q*/*πRₙ*²*γ =* 14.4 in the non-limiting experimental example 4 shown later. Here *Rₕ* is the hydraulic radius of the extrusion channel at a specific position, *µₛ* the shear viscosity of the plasticized matrix, *µ_{s,n}* the shear viscosity at the shear strain rate in the plasticized matrix at the exit port (as estimated in the non-limiting experimental example 10), and *γ* the surface tension of the plasticized material (for the material used in the non-limiting experimental example 4 about 0.04 N/m).

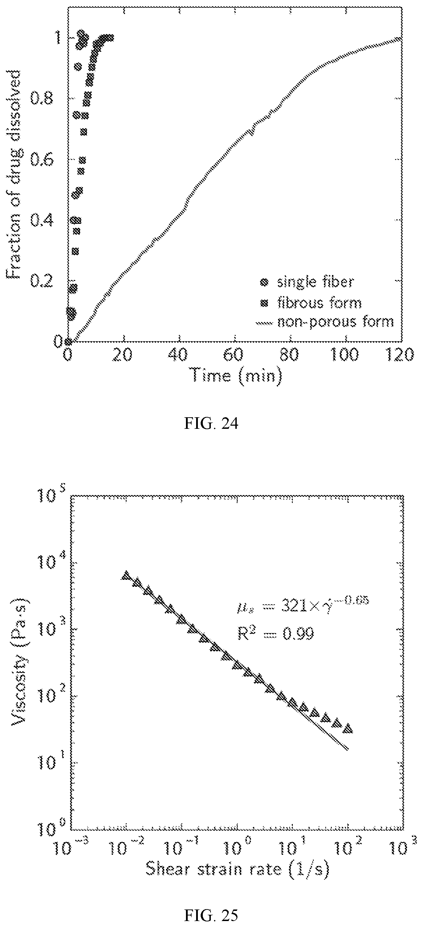

At the values of the Reynolds and Capillary numbers calculated above, inertial and capillary forces are negligible small compared with the viscous forces. Pressure gradients in the extrusion channel are therefore balanced by viscous forces, which are governed by the shear viscosity of the plasticized material. The material of the non-limiting experimental example 4 is non-Newtonian viscous as shown in FIG. 25 and example 9 later. Its shear viscosity may be expressed by the following power law: ${μ}_{s} = m {\dot{γ}}_{s}^{n - 1} 0.01 {s}^{- 1} \leq {\dot{γ}}_{s} \leq 100 {s}^{- 1}$ (7) where *m* and *n* are constants, and *γ̇ₛ* is the shear rate applied.

For small taper angles the pressure gradient may be adapted from the solution to the mass- and momentum balance equations in a circular tube with constant radius to give the following term for the pressure drop, *Δp,* from D to F: $Δ p = \frac{2 {\left(1/n+3\right)}^{n} {mQ}^{n}}{3 {π}^{n} n tan α} \left(\frac{1}{{R}_{n}^{3 n}}-\frac{1}{{R}_{ba}^{3 n}}\right) + \frac{2 {\left(1/n+3\right)}^{n} {mQ}^{n} {L}_{n}}{{π}^{n} {R}_{n}^{3 n + 1}}$ (8) where *Lₙ* is the length of the nozzle of the extrusion channel with constant diameter as shown in FIG. **8**. For the parameter values representative of the non-limiting experimental examples 2 and 4 shown later, *α* = 10.75°, *n* = *0.35, m* = 321, *Rₙ* = 250 µm, *R_{ba} =* 5 mm, *Q* = 2.83 mm³/s, and *Lₙ =* 5 mm, the estimated pressure drop *Δp =* 0.12 MPa. This pressure was developed by the apparatus of the non-limiting experimental example 2 at screw rotation rates of less than about 3-5 rpm.

For further models or examples of fluid flow and pressure drop in a channel, *see e.g.,* R.B. Bird, W.E. Stewart, E.N. Lightfoot, "Transport phenomena", 2nd edn., John Wiley & Sons, 2002. Any more examples of models of the fluid flow and pressure drop (or pressure gradient) in the extrusion channel would be obvious to a person of ordinary skill in the art. Moreover, further models or examples of extruding a plasticized matrix through an exit port to form a plasticized fiber would be obvious to a person of ordinary skill in the art.

### (f) Kinematics of 3D-micro-patterning

The plasticized fiber effluent from an exit port of the extrusion channel is then patterned on a substrate along a deterministic path set by the relative motion of the substrate to the exit port. It may be noted that in the invention herein, the terms "patterning", "micro-patterning", "depositing", "3D-micro-patterning", and "3D-patterning", are used interchangeably. Furthermore, the term "substrate" is referred to a material having a surface on which one or more fibers can be deposited. This includes, but is not limited to a stage (e.g., a working platform on which the fibers can be deposited but which is not part of the dosage form), a deposited fiber bed (which may or may not be part of the dosage form), a biocompatible film or solid material (which may or may not be part of the dosage form), a coating shell, etc.

During micro-patterning, the substrate or stage velocity in the *x-y* plane, *νₛₜ,* is about equal to the velocity of the effluent plasticized fiber. Thus the time to micro-pattern the dosage form structure, *τₘ*, is about the ratio of the length of the fiber in the micro-patterned structure, *L_{f},* to the stage or stream velocity: ${τ}_{m} = \frac{{L}_{f}}{{ν}_{st}} = \frac{{πR}_{f}^{2} {L}_{f}}{Q}$ (9) where *R_{f}* is the radius of the plasticized fiber and ${L}_{f} = \frac{{A}_{{df}^{n} {pl}^{ϕ} f}}{{πR}_{f}^{2}}$ (10) where *A_{df}* is the projected area of the dosage form, *nₚₗ* the number of planes that are micro-patterned (along the *z-*axis), and *φ_{f}* the volume fraction of fibers in the dosage form.

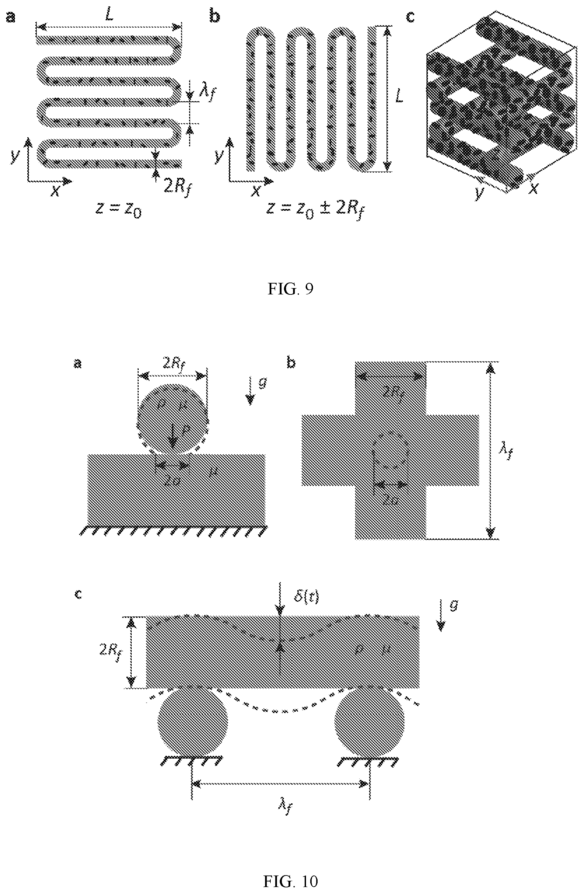

If the fibers in a plane are arranged unidirectionally but perpendicular to the fibers in the planes above and below, as shown in FIGS. **9a****-c,** ${ϕ}_{f} ≅ \frac{π}{2} \frac{{R}_{f}}{{λ}_{f}}$ (11) where *λ_{f}*□ is the inter-fiber spacing.

Thus inserting the parameter values representative of the non-limiting experimental example 4 in Eqs. (10) and (11), *A_{df}* = 100 mm², *nₚₗ =* 10, *R_{f}* = 250 µm, and *λ_{f}* = 900 µm, it follows that *L_{f}* = 1.11 m. Accordingly, if *νₛₜ* = *Q*/π*R_{f}*² *=* 14.4 mm/s, the dosage form is micro-patterned in *τₘ* = 77 seconds.

It may be obvious to a person of ordinary skill in the art that faster process rates can be achieved by increasing the stage velocity (e.g., the velocity at which the fiber exits the exit port) and by increasing the number of exit ports through which a plasticized matrix can be extruded to form one or more plasticized fibers. More examples or models of the kinematics of 3D-micro-patterning would be obvious to a person of ordinary skill in the art.

### (g) Semi-width of fiber-to-fiber contact

The fibers must be deformable to deposit or micro-pattern them along the desired path and to bond them to the fibers below. However, if the fibers deform too much and merge before they are dried, the fibrous patterns are lost and the structural integrity of the dosage form is compromised. Thus, the deformation rate of the fibers at and between the inter-fiber contacts should be controlled.

The contact between two crossed cylindrical fibers, as shown in FIGS. 10a and 10b, may be assumed circular, analogous to that of a sphere on a flat plate. If the stress-strain rate relationship of the viscous fibers is linear and viscous deformation at the contact described by an adapted form of the corresponding elastic solution, the contact radius may be approximated by: $a = {\left(\frac{{πρgR}_{f}^{3} {λ}_{f} {n}_{f} t}{4 {μ}_{s}^{*}}\right)}^{1 / 3}$ (12) where *ρ* is the density of the plasticized fibers, *n_{f}* the number of fibers above the contact, *t* the time after inception of the contact, and *µₛ*^{*} the composite shear viscosity.

Thus for the parameter values representative of the non-limiting experimental example 4, *ρ* = 1000 kg/m³, *R_{f}* = 250 µm, *λ_{f}* = 900 µm, *n_{f}* = 2, and *µₛ*^{*} = 690 Pa·s, by Eq. (12) *a* = 171 µm at *t = τ_{def}* = 15.6 s (*τ_{def}* is the time during which the fiber-to-fiber contact is deformable as estimated in the non-limiting experimental example 6). The calculated value of *a* is slightly smaller than the fiber radius. Thus in the non-limiting example presented, the fibers are expected to bond to each other, while the fibrous microstructure is preserved during drying.

It may be noted that in addition to the deformation at the contact, a viscous fiber also bends downward between the contacts (FIG. **10c**). The maximum deflection, however, is much smaller than the fiber radius for the conditions of the non-limiting experimental examples presented later. Thus bending between the contacts may not be a concern.

From Eqs. (4), (5c), and (12), however, it is readily seen that a critical parameter to ensure that a plasticized matrix is shapeable to a fiber that bonds to the fibers below, but the micro-patterned structure deforms slowly during drying, is the viscosity of the plasticized matrix or wet fiber. As detailed in the experimental example 9, the viscosity of a plasticized material or wet fiber can be controlled, for example, by the weight fraction of solvent in the material or fiber. Thus the amount of solvent added (e.g., the volumetric flow rate at which solvent is injected into the extrusion channel) must be large enough so that the plasticized matrix is readily deformable and the wet fibers bond to each other well during micro-patterning. If the amount of solvent added is too large, however, the micro-patterned structure deforms excessively prior to solvent removal and the fibrous microstructure is lost.

Further models of the deformation of fibers at the contact can be found, for example, in K.L. Johnson, "Contact mechanics", Cambridge University Press, 1985. For models of bending of beams between contacts, *see e.g.,* J.M. Gere, S.P. Timoshenko, "Mechanics of Materials", fourth edn., PWS Publishing Company, 1996. Any more models or examples of the deformation of plasticized fibers in a micro-patterned fibrous structure would be obvious to a person of ordinary skill in the art.

### (h) Drying of a single fiber

After (and during) micro-patterning, the solvent is removed from the fibers. An in-depth analysis of solvent evaporation from a plasticized polymeric fiber must consider a coupled diffusion-convection problem, which takes into account the local dynamics and effects of structural changes of the polymer due to solvent desorption. Such an analysis, however, requires the use of numerical methods and is well beyond the scope of this disclosure.

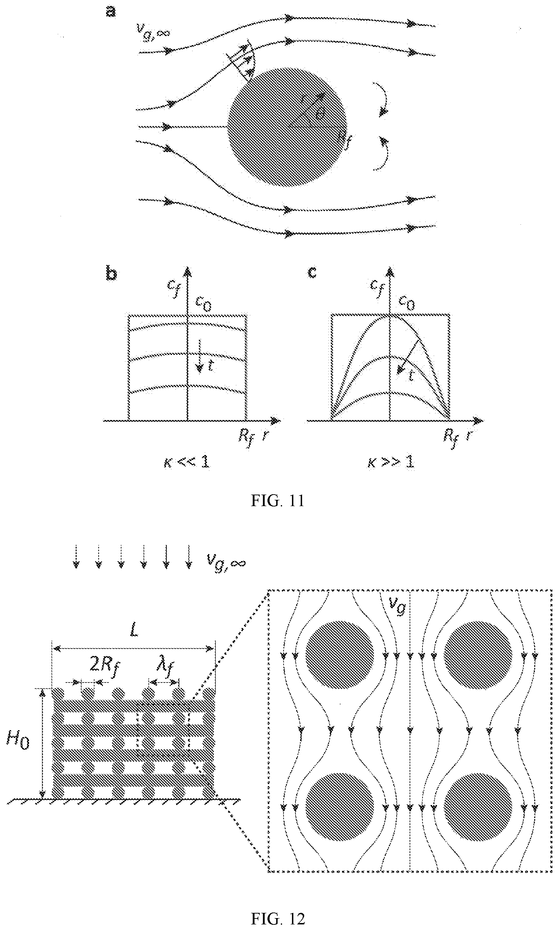

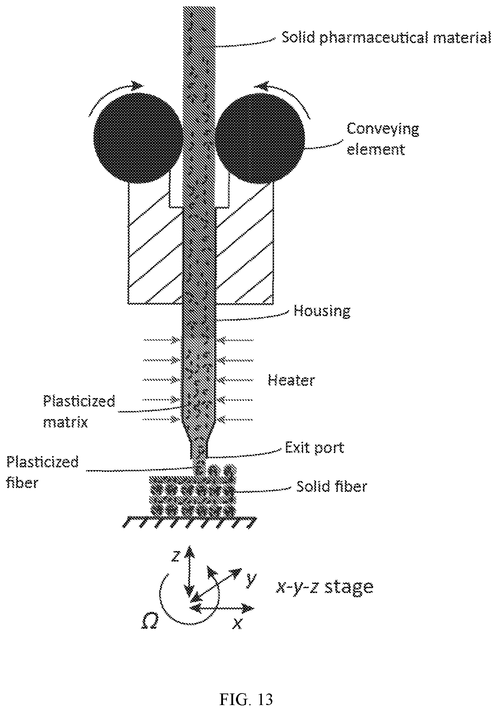

The aim here is modest in that it is to present an approximate but accurate enough model for making engineering decisions. For the configuration shown in FIG. 11a, if the fiber radius is assumed constant and solvent diffusion in the polymer is Fickian with constant diffusivity, D, the solvent concentration in the plasticized fiber, *c_{f},* is governed by: $\frac{\partial {c}_{f}}{\partial t} = D \frac{{\partial }^{2} {c}_{f}}{\partial {r}^{2}}$ (13)

Because the solvent evaporates from the fiber surface and the concentration of solvent in the gas far away from the fiber, *c*_{*g*,∞}, is equal to zero, the boundary condition at *r* = *R_{f}* is: $- D \frac{\partial {c}_{f}}{\partial r} = {k}_{c} \left({c}_{g}-{c}_{g , ∞}\right) ≅ {k}_{c} \frac{{p}_{sat}}{{ρ}_{solv}} \frac{{M}_{w}}{RT} {c}_{f} = {k}_{eff} {c}_{f} r = {R}_{f}$ (14) where *k_{c}* is the convective mass transfer coefficient, *c_{g}* the concentration of solvent in the gas, *pₛₐₜ* the saturation vapor pressure of the solvent in the gas, *M_{w}* the solvent's molecular weight, R the universal gas constant, *T* the temperature, and *k_{eff}* the effective convective mass transfer coefficient.

The convective mass transfer coefficient, *k_{c},* is a function of the Sherwood number, *Sh,* the diffusivity of the solvent molecules in the gas phase, *D_{g},* and the fiber radius, *R_{ḟ}*: ${k}_{c} = Sh \frac{{D}_{g}}{2 {R}_{f}}$ (15) where the Sherwood number depends on the empirical parameters *C*₁ and *m*₁, the Reynolds number *Re* = 2*ρ_{g}ν*_{*g,*∞}*R_{f}*/*µ_{g}* (*ρ_{g}* is the density of the gas, *ν*_{*g*,∞} its far-field velocity, and *µ_{g}* its viscosity), and the Schmidt number *Sc* = *µ_{g}*/*ρ_{g}D_{g}*, as: $Sh = {C}_{1} {Re}^{m} {Sc}^{1 / 3}$ (16)

Thus for the parameter values representative of the non-limiting experimental examples 4 and 5 (a wet fiber with water as solvent that is dried with air at 60 °C and *ν*_{*g*, ∞} = 2.3 m/s), *Re* = 60.75, and, by Eqs. (15) and (16), *k_{c}* = 0.25 m/s (using *D_{g}* = 3.14×10⁻⁵ m²/s, *µ_{g} =* 2×10⁻⁵ Pa·s, *ρ_{g}* = 1.06 kg/m³, and *R_{f}* = 250 µm).

For the above problem, the solvent concentration in the plasticized fiber, *c_{f},* may be approximated by: $\frac{{c}_{f} - {c}_{0}}{{c}_{0}} = 1 - {\sum }_{i = 1}^{∞} \frac{2 {κJ}_{0} \left({rβ}_{i}/{R}_{f}\right)}{\left({β}_{i}^{2}+{κ}^{2}\right) {J}_{0} \left({β}_{1}\right)} exp \left(-{β}_{i}^{2}Dt/{R}_{f}^{2}\right)$ (17) where *c*₀ is the initial concentration of solvent in the fiber, the *βᵢ*s are the roots of ${βJ}_{1} \left(β\right) - {κJ}_{0} \left(β\right) = 0$ (18) (*J*₁ and *J*₀ are the Bessel functions of the first kind) and *κ* is a dimensionless measure of the ratio of the mass transfer resistances inside and at the surface of the fiber: $κ = \frac{{k}_{eff} {R}_{f}}{D} = \frac{{p}_{sat}}{{ρ}_{solv}} \frac{{M}_{w}}{RT} \frac{{k}_{c} {R}_{f}}{D}$ (19)

Thus if *κ* << 1, the evaporation rate of the solvent is limited by convection of solvent molecules away from the fiber-gas interface. The solvent concentration in the fiber during drying is roughly constant in this case as shown in FIG. **11b****.** If *κ* >> 1, however, the solvent evaporation rate is mostly limited by the diffusive flux in the fiber and the solvent concentration profile in the fiber is as shown in FIG. **11c****.**

In the non-limiting experimental examples 4 and 5 shown later, *D* = 2.5×10⁻¹⁰ m²/s, *pₛₐₜ =* 20 kPa, *ρₛₒₗᵥ =* 1000 kg/m³, *M_{w}* = 18 g/mol, *T* = 60 °C, *k_{c}* = 0.25 m/s, and *R_{f}* = 250 µm. Thus *κ* = 31.9. At such values of *κ,* the evaporation rate is mostly limited by the diffusive flux of solvent in the fiber.

Thus the ratio of the mass of solvent removed from the fiber at time *t, m*(*t*), to the corresponding quantity at infinite time, *m*_{∞}, can be approximated by: $\frac{m \left(t\right)}{{m}_{∞}} = 1 - {\sum }_{i = 1}^{∞} \frac{4 exp \left(-{β}_{i}^{2}Dt/{R}_{f}^{2}\right)}{{β}_{i}^{2}}$ (20)

From the graphical solution to this equation presented by Crank, 98 percent of the solvent is removed if *Dt*/*R_{f}*² ≈ 0.77. The drying time of a wet fiber may therefore be estimated by:
By way of example but not by way of limitation, if *R_{f}* = 250 µm and *D* = 2.5×10⁻¹⁰ m²/s, by Eq. (21) *τ*_{0.98} = 193 s. The drying time is comparatively short because of the large specific surface area of the thin fibers.

Further models of the diffusion of solvent through polymers can be found, for example, in J. Crank, "The mathematics of diffusion", second edition, Oxford University Press, 1975; and in J. Crank. G.S. Park, "Diffusion in polymers", Academic Press, 1968. Any more models or examples of drying of plasticized fibers would be obvious to a person of ordinary skill in the art.

### (i) Drying of a fibrous dosage form

Within the fibrous dosage form structure, the velocity of the dry gas (or air stream) around the fibers cannot be determined by analytical methods. An approximation may, however, be obtained for a system where the flow in the interior of the fibrous structure is dominated by viscous forces. The streamlines around the fibers in the structure may be as shown in FIG. **12** in this case. The average fluid velocity in the open spaces in the direction of flow, *ν̅_{g}* , may then be approximated by Darcy's law: ${\overline{ν}}_{g} = \frac{1}{1 - {ϕ}_{f}} \frac{K}{{μ}_{g}} \frac{{dp}_{g}}{dx}$ (22) where *K* is the hydraulic permeability and *dp_{g}*/*dx* the pressure gradient of the gas across the fibrous structure.

The hydraulic permeability may be estimated by the Kozeny-Carman equation: $K = \frac{1}{k} \frac{{\left(1-{ϕ}_{f}\right)}^{3}}{{A}_{ν}^{2}}$ (23) where the Kozeny constant, *k* ≈ 5, and the surface area of fibers per unit volume of the dosage form, *A_{ν}* = *π*/*λ_{f}.*

The pressure gradient in the direction of gas flow through the fibrous structure may be estimated as: $\frac{{dp}_{g}}{dx} ≅ \frac{{ρ}_{g} {ν}_{g , ∞}^{2}}{L}$ (24) where *ν*_{*g,*∞} is the far-field velocity of gas in the direction of flow and *L* the cord length of the dosage form structure.

Thus by way of example but not by way of limitation, if *ρ_{g} =* 1.06 kg/m³, *µ_{g}* = 2×10⁻⁵ Pa·s, *ν*_{*g*,∞} = 2.3 m/s, *L* = 5 mm, *φ_{f}* = 0.44, and *Aᵥ* = 3491 1/m, the average gas velocity in the pores (e.g., in the free spaces between the fibers), *ν̅_{g}* = 0.1 m/s. Assuming that the maximum velocity in the pores is a factor two greater than the average velocity (i.e., *ν_{g,max}* = 2 *ν̅_{g}* = 0.2 m/s), the Reynolds number of the gas flow around a fiber in the dosage form, *Re =* 2*ρ_{g}ν_{g,max}R_{f}*/*µ_{g} =* 7.7. At such Reynolds numbers, the boundary layer separates behind the fibers and thus the viscous flow assumption (and Darcy's law) used here to calculate *ν_{g,max}* must be considered highly approximate.

Nonetheless, the estimated value of the Reynolds number may be inserted in Eq. (16), and then the convective mass transfer coefficient may be derived by Eq. (15). For the parameter values shown above and in section (h) (and a far-field velocity around a fiber of 0.2 m/s), *k_{c}* = 0.1 m/s and *κ* = 13.5 >> 1. Thus under the non-limiting conditions presented, the drying time of the fibers in the fibrous structure is limited by the diffusion of solvent through the fibers, too, and can be approximated by Eq. (21).

Further models for estimating the gas velocity in the fibrous structure can be found, for example, in J. Happel and H. Brenner, "Low Reynolds number hydrodynamics with special application to particulate media", Prentice-Hall, Englewood Cliffs, NJ, 1965. Any more models or examples of drying of fibrous structures would be obvious to a person of ordinary skill in the art.

### (k) Contraction of fibers during drying

As solvent is removed from the fibers upon drying, the fibers may contract. Assuming that the plasticized fibers are ideal solutions (i.e., the solid and liquid volumes are conserved upon drying), and that contraction is isotropic, the ratios of radius and inter-fiber distance of the solid fibers, *Rₛ* and *λₛ*, to the corresponding values of the plasticized or fluidic fibers, *R_{f}* and *λ_{f}*, may be approximated as: $\frac{{R}_{s}}{{R}_{f}} = \frac{1}{{ϕ}_{ν}^{1 / 3}} {\left(1-\frac{{c}_{0}}{{ρ}_{solv}}\right)}^{1 / 3}$ (25a) $\frac{{λ}_{s}}{{λ}_{f}} = \frac{1}{{ϕ}_{ν}^{1 / 3}} {\left(1-\frac{{c}_{0}}{{ρ}_{solv}}\right)}^{1 / 3}$ (25b) where *φ_{ν}* is the volume fraction of voids in the solid fibers (the plasticized fibers may in some embodiments be assumed pore-free) and *c*₀ the initial concentration of solvent in the plasticized fibers. In the non-limiting experimental example 4 shown later, *c*₀/*ρₛₒₗᵥ =* 0.4, and thus *Rₛ*/*R_{f}* = *λₛ*/*λ_{f}* ≥ 0.84.

### (l) Summary of models

The above models illustrate the effects of the design of the feeding unit (e.g., the applied control variables), the properties of the input material (e.g., the radius of an excipient particle and the diffusivity of solvent in it), the microstructural parameters of the dosage form (e.g., the fiber radius and inter-fiber spacing), the properties of the plasticized material (e.g., its viscosity and density (both are affected by the weight or volume fraction of solvent in it), and the diffusivity of solvent in it), and additional process parameters (e.g., the screw rotation rate and geometry, and the velocity and temperature of the dry gas stream) on the composition, microstructure, and process time of the fibrous dosage forms. It is demonstrated that the composition and microstructure of the fibrous forms can be precisely controlled by the method and apparatus disclosed herein, while the process time can be kept short.

It may be obvious to a person of ordinary skill in the art that many more examples and models of wet-processing fibrous structures and fibrous dosage forms could be developed and presented. Similarly, it is obvious to a person of ordinary skill in the art that models and examples could be presented for producing melt-processed fibrous structures and dosage forms.

### Elements of the Method and Apparatus

In view of the theoretical considerations and examples above, which are suggestive and approximate rather than exact, the aspects and embodiments of the present invention may further include the following elements.

In any aspect of the method and apparatus disclosed herein, the geometry of the extrusion channel cross section can assume any shape, and thus may be circular, elliptical, polygonal, combinations thereof, etc. Furthermore, the extrusion channel cross section may be uniform or non-uniform along its length. Thus, in some embodiments the extrusion channel cross section tapers down before an exit port to the cross section of said exit port. Moreover, in some embodiments the extrusion channel bifurcates into at least one other end comprising an exit port. Multiple exit ports are desirable for achieving high process rates.

In some embodiments of the aspects disclosed herein, a fraction of the housing is optically transparent. Said fraction is between 0 and 1 in the disclosure herein (e.g, the fractions 0 and 1 are included). A partially or entirely optically transparent housing may enable improved optical sensing of the state or structure of the material (e.g., the one or more solid constituents or the plasticized matrix) in the extrusion channel. Non-limiting examples of materials which an optically transparent fraction of the housing may consist of include one of glass (e.g., silicon dioxide, borosilicate, alumina, germanium dioxide, spectrosil quartz, etc.) or plexiglass (e.g., poly(methyl methacrylate)). Non-limiting examples of materials which a non-optically-transparent fraction of the housing may consist of include one of metals, such as various forms of steel, stainless steel, iron, aluminum, tungsten, iridium, nickel, platinum, copper, or alloys or combinations thereof, etc., or one of polymers such as polyethylene, polypropylene, polystyrene, polycarbonate, acrylonitrile butadiene styrene, etc.

A conveying element in the invention herein comprises a device that transports a fluid (e.g., a liquid, plasticized material, etc.) or a solid (e.g., a solid filament, solid sheet, granular solid, etc.) towards an exit port of the extrusion channel by applying mechanical work on said fluid or solid. Thus, non-limiting examples of conveying elements for applying mechanical work on a solid or fluid in the extrusion channel include fluid pumps (e.g., peristaltic pumps, diaphragm pumps, rotary vane pumps, extrusion gear pumps, etc.), pistons, extrusion or conveyor screws, pressurized gas or liquid, piezoelectric actuators, etc. A conveying element may be operated mechanically (e.g., hydraulically, pneumatically, etc.) or with a motor (e.g., an electrical AC motor, DC motor, stepper motor, etc.), among others.

It may be noted that a screw is a preferred conveying element herein. By way of example but not by way of limitation, a screw enables to integrate such process steps as conveying granular solid towards and exit port, mixing drug and excipient (and solvent), forming a plasticized matrix by heating or solvation of one or more solid constituents, conveying a plasticized matrix towards an exit port of the extrusion channel, and so on into an efficient, continuous process.

The apparatus or method disclosed herein may further comprise a solids feeding unit for feeding or injecting one or more solid constituents into the extrusion channel. By way of example but not by way of limitation, said solids feeding unit may comprise a device that is capable of controlling the rate at which one or more solid constituents are injected into the extrusion channel by either volumetric or gravimetric control. The device may be operated in a continuous (e.g., one or more solid constituents are continuously injected into the extrusion channel), semi-continuous, or batch mode (e.g., specific volumes of one or more solid constituents are injected into the extrusion channel at specific times). Furthermore, the one or more solid constituents may be injected in the form of one or more granular solids as in the schematics of FIGS. 3, 4, and 6, one or more filaments, one or more sheets, or any other geometry. Another non-limiting example of a solids feeding unit is a hopper filled with one or more solid constituents in the form of one or more granular solids. The hopper may permit flow of the one or more granular solids from the hopper into the extrusion channel. The flow of said granular solids may, for example, be driven by gravity. Any more examples of solids feeding units would be obvious to a person of ordinary skill in the art.

In some embodiments, a fraction of (or all) the input material is supplied to the extrusion channel in the form of a plasticized matrix. Thus, the apparatus or method disclosed herein may further comprise a plasticized matrix feeding unit for feeding or injecting a plasticized matrix into the extrusion channel. By way of example but not by way of limitation, said plasticized matrix feeding unit may comprise a device that is capable of controlling the rate at which a plasticized matrix is injected into the extrusion channel by either volumetric or gravimetric control. The device may be operated in a continuous (e.g., a plasticized matrix is continuously injected into the extrusion channel), semi-continuous, or batch mode (e.g., specific volumes of a plasticized matrix are injected into the extrusion channel at specific times). Any more examples of a plasticized matrix feeding unit would be obvious to a person of ordinary skill in the art.

In some embodiments, at least one input constituent is solid but plasticizes by solvation upon contact with a suitable solvent. Thus, the apparatus disclosed herein may further comprise a solvent feeding unit attached to a feeding port for injecting at least one solvent into the extrusion channel. A solvent feeding unit comprises any device to move/dispense/inject solvent at a controlled rate into the extrusion channel, such as a peristaltic pump, a diaphragm pump, a rotary vane pump, a syringe pump, or any other rotary or positive displacement pump, among others.

Also, in some embodiments at least one input constituent is solid but plasticizes by melting upon heating to a temperature above its melting temperature. Thus, the apparatus herein may further comprise at least one heating element. Said at least one heating element may, for example, be wrapped around the housing, such as a band heater, coil heater, etc. that surrounds a fraction (or all) of the housing's outer surface. At least one heating element may also be embedded into the housing, such as one or more cartridge heaters fixed in and surrounded by the housing, among others. Furthermore, at least one heating element may be partially embedded into the housing, such as fluid channels in the housing filled with a circulating fluid, said circulating fluid being temperature-controlled by an external temperature control unit. Such a system may not only permit heating, it may also allow to cool the housing if necessary and thereby enable improved temperature control. It may be noted that in the context of this disclosure, all such heating elements that are "partially embedded into the housing" are considered "embedded into the housing". Thus, in some embodiments of the method disclosed herein, the heating may be performed using one or more wrap around heaters, or one or more heaters embedded into the housing, or one or more furnaces, or any combinations thereof, among others. Any more examples of heating elements or examples of how the heating of the housing or an input constituent may be performed would be obvious to a person of ordinary skill in the art.

In some embodiments of the apparatus or method herein, the housing further comprises at least one sensing port for attaching a sensor to the housing. Said sensor may be selected from the group comprising pressure sensors, temperature sensors, flow rate sensors, sensors for measuring the composition of the material in the extrusion channel (e.g., by near infrared spectroscopy, Fourier transform infrared spectroscopy, nuclear magnetic resonance spectroscopy, raman spectroscopy, etc.), or sensors for determining the physical form of the material in the extrusion channel (e.g., by X-ray spectroscopy, Fourier transform infrared spectroscopy, nuclear magnetic resonance spectroscopy, raman spectroscopy, etc.), and others.

A sensor may, for example, be used to monitor a specific variable or property, or to provide a measured signal for real-time feedback control of a specific variable. Such real-time feedback control is desirable for continuous manufacturing with integrated, real-time quality control. Thus, in some embodiments of the apparatus or method herein, at least one sensor supplies a measured signal to a control loop comprising at least a reference signal, an actuator, and a sensor. By way of example but not by way of limitation, if real-time feedback control of the rate at which a plasticized matrix is extruded through an exit port is aimed for, the sensor may comprise a pressure or flow rate sensor, the actuator may be a conveying element, and the reference signal may be a target flow rate or pressure.

In some embodiments of the apparatus or method herein, the housing further comprises at least one other feeding port for injecting fluid into the extrusion channel. Said fluid may, for example, be attached to (or surround) a plasticized or solid fiber, or it may be embedded in a fiber. In some embodiments, the fluid solidifies after injection into the extrusion channel. Thus if the fluid is attached to a fiber initially, it may form a solid coating of said fiber after solidification.

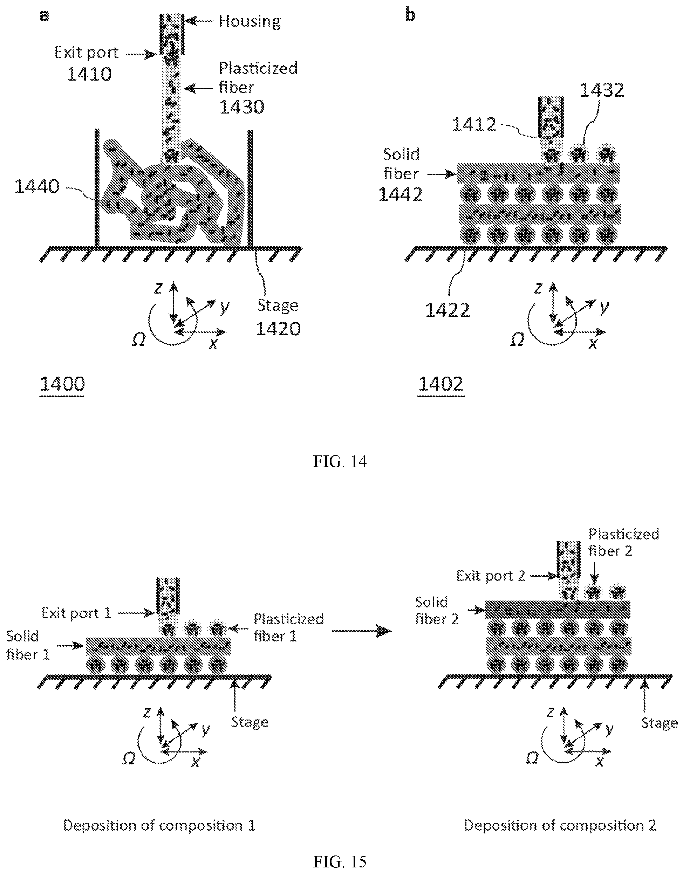

FIG. **14** presents illustrative but not limiting examples **1400, 1402** of an exit port **1410, 1412** and a fiber structuring unit comprising a moving stage **1420, 1422** to build (e.g., micro-pattern or 3D-pattern) fibrous dosage forms or three dimensional structural networks of one or more drug-containing fibers. In the context of the invention herein, a stage **1420, 1422** comprises a working platform that does not become part of the dosage form. By way of example but not by way of limitation, said working platform or stage comprises a grid defining a deposition surface on which a plasticized fiber can be deposited. According to the claimed invention, the fiber structuring unit comprises a stage that is movable with respect to an exit port (e.g., this includes, but is not limited to a stage that is movable and an exit port that is not movable, or an exit port that is movable and a stage is not movable, or an exit port that is movable and a stage that is movable). Similarly, according to the claimed invention, structuring at least one plasticized fiber to a fibrous dosage form is performed using a stage that is movable with respect to an exit port. Furthermore, in the apparatus or method herein, one or more plasticized fibers may be structured to at least a fraction of a fibrous dosage form by 3D-patterning said one or more plasticized fibers on a substrate defined by a stage or attached to a stage. It may be noted that the terms "stage" and "*x-y-z* stage" are used interchangeably in this disclosure.

FIG. **14a** shows a non-limiting schematic **1400** of structuring one or more plasticized fibers **1430** to a three dimensional structural network of one or more drug-containing fibers by randomly (or almost randomly) arranging/assembling (or 3D-patterning) said one or more plasticized fibers **1430** on a substrate defined by a stage **1420** or attached to a stage **1420.** The random (or almost random) arrangement/assembly is achieved because the distance between the exit port **1410** and the location where a plasticized fiber **1430** effluent from the exit port **1410** is deposited is so long that the plasticized fiber **1430** can bend randomly (or almost randomly) before deposition. It may be noted that in the non-limiting example shown, the deposition location of a plasticized fiber lies on the deposition surface of the stage **1420** initially, but then after an initial layer of fibers **1440** has been deposited on the stage **1420,** the deposition location of a plasticized fiber lies on the deposited layer of fibers **1440.**

FIG. **14b** presents another non-limiting schematic **1402** of structuring one or more plasticized fibers **1432** to a three dimensional structural network of fibers by 3D-patterning said one or more plasticized fibers **1432** on a substrate defined by a stage **1422** or attached to a stage **1422.** In this schematic, the distance between the exit port **1412** and the deposition location of a plasticized fiber **1432** effluent from said exit port **1412** is small and controlled during 3D-patterning. Thus the one or more plasticized fibers **1432** effluent from the exit port **1412** do not bend randomly (or almost randomly) before deposition in this case. Therefore, the deposition location of the one or more plasticized fibers **1432** effluent from the exit port **1412** can be precisely controlled as shown.

In some embodiments of the method or apparatus herein, accordingly, for achieving precise control of the deposition location of a fiber, the distance between an exit port and the deposition location of a plasticized fiber effluent from said exit port is no greater than 7 mm during 3D-patterning. This includes, but is not limited to a distance between an exit port and the deposition location of a plasticized fiber effluent from said exit port no greater than 6 mm, or no greater than 5 mm, or no greater than 4 mm, or no greater than 3 mm, or no greater than 2 mm. Furthermore, in some embodiments the distance between an exit port and a deposition location of a plasticized fiber on a substrate is no greater than ten times the thickness of said fiber. This includes, but is not limited to a distance between an exit port and a deposition location of a fiber on a substrate no greater than 9 times, or no greater than 8 times, or no greater than 7 times, or no greater than 6 times, or no greater than 5 times the thickness of a fiber. It may be noted that the deposition location of a plasticized fiber can be the surface of a substrate (e.g., the top surface of an *x-y-z* stage or the top surface of a deposited fibrous bed or structure).

Moreover, for achieving precisely controlled fibrous patterns, the velocity of a substrate with respect to an exit port may be of the order of the velocity of a fibrous extrudate effluent from an exit port (e.g., the velocity of a plasticized fiber that exits an exit port). Thus, in some embodiments the velocity of a substrate with respect to an exit port, *νₛₜ,* is in the range 0.1-10 times the velocity of a fibrous extrudate, *ν_{f}*. This includes, but is not limited to *νₛₜ* in the range 0.2-5 times *ν_{f}*, or *νₛₜ* in the range 0.3-3 times *ν_{f}*, or *νₛₜ* in the range 0.5-2 times *ν_{f}.* It may be noted that instead of or in addition to a substrate, an exit port may be moved to achieve precise control of the location where a fiber is deposited. Moreover, it may be obvious to a person of ordinary skill in the art that the path and velocity of a substrate with respect to an exit port may be computer-controlled.

In addition to the requirements on the kinematics of a substrate with respect to an exit port, a plasticized fiber must be viscous enough to ensure that a precise fibrous pattern is preserved. Thus, in some embodiments the shear viscosity of a plasticized matrix or fiber is greater than 0.1 Pa·s at a shear rate no greater than 10 1/s. This includes, but is not limited to a shear viscosity of a plasticized matrix or fiber greater than 0.5 Pa·s, or greater than 1 Pa·s, or greater than 5 Pa·s, or greater than 10 Pa·s, or greater than 20 Pa·s, or greater than 50 Pa·s, or greater than 100 Pa·s at a shear rate no greater than 10 1/s.

In some embodiments, the viscosity of a plasticized fiber is controlled by the weight fraction of solvent in said fiber. Thus, in some embodiments, the weight fraction of solvent in a plasticized fiber is no greater than 0.925. This includes, but is not limited to a weight fraction of solvent in a plasticized fiber no greater than 0.9, or no greater than 0.85, or no greater than 0.8, or no greater than 0.75, or no greater than 0.7, or no greater than 0.65, or no greater than 0.6.

Under consideration of the above theory, models, and requirements on process parameters and material properties, an inter-fiber spacing, and/or a fiber thickness, and/or the position of an inter-fiber contact, and/or a contact width of an inter-fiber contact can be precisely (or deterministically) controlled in a fibrous dosage form (or a three dimensional structural network of fibers) prepared by the method or apparatus of this disclosure. In the context of the invention herein, a variable (or a parameter, e.g., an inter-fiber spacing and a fiber thickness) is precisely controlled if it is deterministic and not stochastic (or random). A variable or parameter may be deterministic if, upon multiple repetitions of a step that includes said variable, the standard deviation of the values of said variable is smaller than the average value. This includes, but is not limited to a standard deviation of the values of said variable smaller than half the average value, or smaller than one third of the average value, or smaller than a quarter of the average value, or smaller than one fifth or the average value, or smaller than one sixth of the average value of said variable. By way of example but not by way of limitation, if a fiber is produced multiple times under identical conditions, the standard deviation of the thickness of said fibers is less than the average value of said fibers' thickness. Similarly, if an inter-fiber spacing is produced multiple times under identical conditions, the standard deviation of said inter-fiber spacing is less than the average value of said inter-fiber spacing in some embodiments of the invention herein.

After patterning on a substrate, a plasticized fiber may be solidified. By way of example but not by way of limitation, depending on the composition of said plasticized fiber, solidification may be by removing solvent from the plasticized fiber, by cooling the plasticized fiber to below its melting temperature, or by crosslinking some of the constituents of the plasticized fiber.

To accelerate the rate at which solvent is removed from a fiber, or to accelerate the rate at which some of the constituents of a fiber crosslink, the apparatus herein may further comprise a unit for blowing warm air or gas on the fibrous structure. Similarly, to accelerate the rate at which a fiber is cooled, the apparatus herein may further comprise a cooling unit for blowing cool air or gas on the fibrous structure. The far-field velocity of the gas may be greater than 0.1 m/s in some embodiments herein. This includes, but is not limited to a far-field velocity of the gas greater than 0.2 m/s, or greater than 0.5 m/s, or greater than 1 m/s.

Also, to accelerate the rate at which solvent is removed from a fiber, or to accelerate the rate at which some of the constituents of a fiber crosslink, in some embodiments the stage on which the fibrous structure is deposited may comprise a grid that is impermeable to the deposited fiber but permits air or gas flow through it. The stage on which the fibrous structure is deposited may further be temperature-controlled.

As shown in the illustrative but not limiting example of FIG. **15****,** any apparatus or method disclosed herein may be adapted to manufacture fibrous dosage forms comprising multiple fibers of different compositions. By way of example but not by way of limitation, plasticized fiber 1 with composition 1 may first be extruded through exit port 1 and the plasticized fiber 1 deposited on a substrate. Then plasticized matrix 2 with composition 2 may be extruded through exit port 2 and the plasticized fiber 2 deposited on a substrate. Furthermore, an exit port through which a plasticized matrix is extruded may be surrounded by another exit port through wich another plasticized matrix is extruded. Fibers with heterogeneous composition of the cross section may be produced that way.

The fibrous dosage forms produced by some embodiments of the apparatus or method herein disintegrate in a time no greater than 45 minutes after immersion in a dissolution fluid. This includes, but is not limited to a disintegration time no greater than 30 minutes, or no greater than 25 minutes, or no greater than 20 minutes, or no greater than 15 minutes, or no greater than 10 minutes after immersion in a dissolution fluid.

To achieve such disintegration times, in some embodiments the one or more fibers have an average thickness *h*₀ no greater than 2.5 mm. This includes, but is not limited to *h*₀ no greater than 2 mm, or no greater than 1.5 mm, or in the ranges of 0.1 µm to 2.5 mm, 0.5 µm to 2.5 mm, 1 µm to 2.5 mm, 1.75 µm to 2.5 mm, 2.5 µm to 2.5 mm, 2.5 µm - 2 mm, 5 µm - 2.5 mm, 10 µm - 2.5 mm, 15 µm - 2.5 mm, 20 µm - 2.5 mm, 30 µm - 2.5 mm, 40 µm - 2.5 mm, or 50 µm - 2.5 mm.

Furthermore, in some embodiments, the contact width, 2*a*, between fibers (or fiber segments) on average is no greater than 2.5 mm. This includes, but is not limited to a contact width between fibers no greater than 2 mm, or no greater than 1.75 mm, or no greater than 1.5 mm. In other examples without limitation, a contact width, 2*a*, between fibers may be no greater than 1.1 times the thickness of the contacting fibers (or fiber segments) at the position of the contact.

Moreover, in some embodiments the effective free spacing, *λ_{f,e},* on average is greater than 0.1 µm. This includes, but is not limited to an average *λ_{f,e}* greater than 0.25 µm, or greater than 0.5 µm, or greater than 1 µm, or greater than 2 µm, or greater than 5 µm, or greater than 7 µm, or greater than 10 µm, or greater than 15 µm, or greater than 20 µm, or greater than 25 µm, or greater than 30 µm, or greater than 40 µm, or greater than 50 µm, or in the ranges of 0.1 µm - 5 mm, 0.1 µm - 3 mm, 0.25 µm - 5 mm, 0.5 µm - 5 mm, 0.25 µm - 3 mm, 0.1 µm - 2.5 mm, 1 µm - 2.5 mm, 5 µm - 2.5 mm, 10 µm - 2.5 mm, 15 µm - 3 mm, 20 µm - 3 mm, 30 µm - 3 mm, 40 µm - 3 mm, or 50 µm - 3 mm. The "effective free spacing" between adjoining fiber segments is defined as the maximum diameter of a sphere that fits in the corresponding free space considering the fibers as rigid, fixed bodies. The diameter of such spheres may be estimated from 2-d images of the microstructure. Such 2-d images may be obtained from scanning electron micrographs of the cross section of the dosage form. The greatest circles that fit in the free spaces of the microstructure may be drawn on the scanning electron micrograph (e.g., the 2-d image) and the average diameter of the circles (e.g., the average effective free spacing) calculated. It may be noted that in the context of the invention herein, the average effective free spacing (e.g., the effective free spacing on average) is referred to a volume-average, or area-average, or line-average effective free spacing rather than a number-average effective free spacing. The above constraints on the effective free spacing are primarily for ensuring that dissolution fluid can percolate into and flow through the fibrous structure at moderate velocity. This enables that the disintegration time of the "thick" dosage form is of the order of the disintegration time of a "thin" single fiber under the given flow conditions.

Further details related to the disintegration of and drug release by fibrous dosage forms, and the definitions of *h*₀, *a,* and *λ_{f,e}* can be found in the U.S. patent application Ser. No. 15/482,776.

### EXPERIMENTAL EXAMPLES

The following examples set forth, in detail, ways by which the fibrous dosage forms may be prepared and analyzed, and will enable one of skill in the art to more readily understand the principle thereof. The following examples are presented by way of illustration and are not meant to be limiting in any way.

### Example 1: Apparatus for preparing melt-processed single fibers and dosage forms

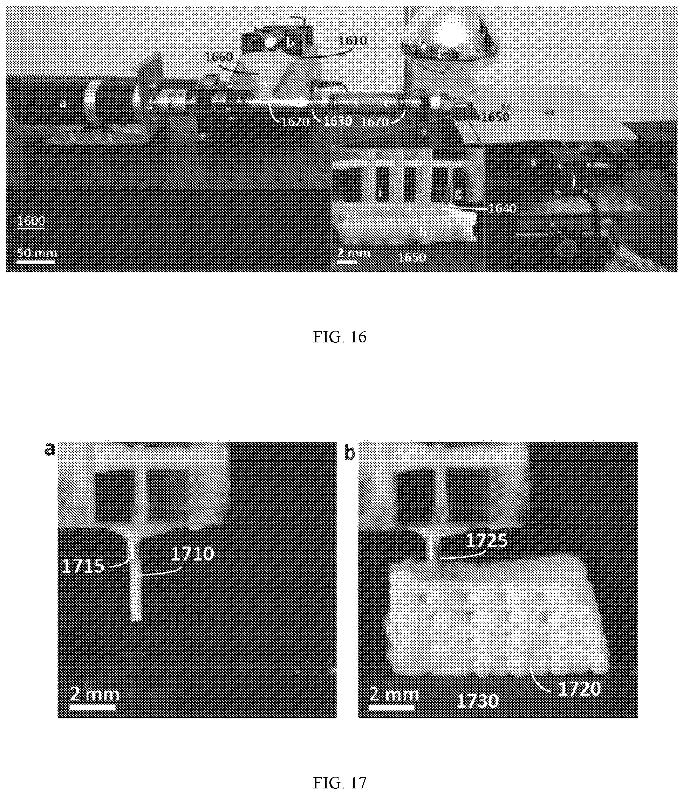

FIG. **16** is a photograph of an apparatus **1600** to prepare melt-processed fibrous dosage forms. It includes the following elements or units: a granular solid feeding unit **1610,** a housing **1630** defining an extrusion channel, an extrusion screw **1620** (i.e., the screw **1620** is inside the housing **1630**), a wrap around and an infrared heater, an exit port **1640** of the extrusion channel where the fibrous melt exits, and an *x-y-z* stage **1650** for building the fibrous structure.

The granular solid feeding unit **1610** consists of a syringe with uniform barrel diameter and a syringe pump to inject one or more granular solids into a hopper **1660.** The volumetric flow rate is controlled by velocity control of the syringe's piston. The hopper **1660** directs the granules into the extrusion channel through a granular solids feeding port in the housing. The material (e.g., the one or more granular solids) in the extrusion channel is conveyed forward by the rotating extrusion screw **1620.** The extrusion screw **1620** is 244 mm long, has an outer diameter of 10 mm, a helix angle of 17.65°, a screw channel height of 1 mm, and a screw channel width of 8.5 mm. The housing is surrounded by a resistance heater coil **1670** to set the temperature of the housing **1630** and extrusion channel. The radius of the extrusion channel is uniform and equal to 5 mm at the location of the screw, but it tapers down before the exit port to the radius of said exit port. The radius of the exit port (e.g., the nominal radius of a plasticized fiber, *Rₙ*, extruded through the exit port) is 0.25 mm. The *x-y-z* stage **1650** is positioned right underneath the exit port.

### Example 2: Apparatus for preparing wet-processed single fibers and dosage forms

The apparatus presented in the non-limiting experimental example 1 above may adapted for preparing wet-processed single fibers and dosage forms. Specifically, in the non-limiting experimental examples herein, a second feeding port for injecting at least one solvent into the extrusion channel is added to the housing between the granular solids feeding port and the exit port. Also, a solvent feeding unit attached to the second feeding port is added. The solvent feeding unit consists of a syringe with uniform barrel diameter and a syringe pump to control the volumetric flow rate at which solvent is injected into the extrusion channel. Additionally, in the non-limiting experimental examples herein, a temperature-controlled fan is added to the apparatus shown in the experimental example 1 above to blow warm air on the plasticized micro-patterned fibers for accelerating solvent removal from the fibrous structure.

### Example 3: Preparation of melt-processed single fibers and dosage forms

Melt-processed single fibers, fibrous dosage forms, and minimally-porous (or non-porous) dosage forms were prepared by first mixing 40 wt% of solid acetaminophen particles with 60 wt% polyethylene glycol 35,000 (PEG 35k) granules. The solid mixture was then loaded into the granular solid feeding unit set to deliver 1.7 mg/s. The rotation rate of the screw was about 3-5 rpm and the temperature of the housing was set to 80 °C.

For preparing melt-processed single fibers and fibrous dosage forms, the fibrous melt effluent from the exit port of the extrusion channel was deposited along the desired path on the *x-y-z* stage. The ambient and stage temperatures were 25 °C during deposition, thus the deposited fiber bed cooled and solidified soon after it was formed. A photograph of a fiber bed during fiber deposition (or 3D-micro-patterning) is shown in the magnified window of FIG. **16****.** Six different structures were prepared: single fibers with nominal radius, *Rₙ* = 250, 500, and 1000 µm (as given by the radius of the exit port), and fibrous assemblies (e.g., fibrous dosage forms) of the configuration shown in FIG. **1b** with *Rₙ* = 250 µm and nominal inter-fiber spacing, *λₙ* = 1750, 900, and 600 µm (as determined by the path of the *x-y-z* stage). The velocity of the stage was 7.3 mm/s while the fiber and fibrous forms with *Rₙ* = 250 µm were deposited, and 1.8 and 0.45 mm/s, respectively, for depositing the fibers with *Rₙ* = 500 and 1000 µm. Also, during deposition the distance between the exit port and the top of the stage or fibrous bed was kept at 1 -3 mm. The process was stopped when the single fiber was deposited, or, in case of fibrous dosage forms, the thickness of the fibrous bed reached about 5 mm. The melt-processed fibrous dosage forms prepared were square disks: 10 mm in side length and 5 mm in thickness.

For preparing melt-processed minimally-porous solid dosage forms, a stainless steel mold was placed on (e.g., attached to) the *x-y-z* stage and was filled with the plasticized fibrous stream effluent the exit port until a height of about 5 mm was reached. The temperature of the mold was 25 °C. The material was left in the mold for about 2 minutes to solidify and the solid dosage form was subsequently ejected. The melt-processed non-porous dosage forms prepared were circular disks: 13 mm in diameter and 5 mm in thickness.

### Example 4: Preparation of wet-processed single fibers and dosage forms

Wet-processed single fibers and fibrous and minimally-porous dosage forms were prepared by first mixing 60 wt% of solid ibuprofen particles with 40 wt% particles of polyvinyl alcohol (PVA)-polyethylene glycol (PEG) graft copolymer 3:1 of molecular weight 45 kg/mol (tradename Kollicoat IR, BASF, Ludwigshafen, Germany). The particles were mixed and loaded into the granular solid feeding unit set to deliver 1.7 mg/s. The solvent feeding unit was filled with DI water and the volumetric flow rate of water was 1.13 mm³/s. The rotation rate of the extrusion screw was about 3-5 rpm.

For preparing wet-processed single fibers and fibrous dosage forms, the plasticized fibrous effluent from the exit port of the extrusion channel was deposited along the desired path on the *x-y-z* stage. Two different structures were prepared: single fibers with nominal radius, *Rₙ* = 250 µm (as given by the radius of the exit port), and fibrous assemblies (e.g., fibrous dosage forms) of the configuration shown in FIG. **1b** with *Rₙ* = 250 µm and nominal inter-fiber spacing, *λₙ* = 900 µm (as determined by the path of the *x-y-z* stage). The velocity of the stage was 14.4 mm/s during deposition of the fiber and fibrous dosage form. The distance between the exit port and the deposition location of a plasticized fiber effluent from said exit port (e.g., the top surface of the deposited fiber bed or the top surface of the stage initially) was kept at 1 to 2 mm. The process was stopped when the single fiber was deposited or, in the case of fibrous dosage forms, 10 fibrous planes were patterned. During and after patterning, warm air at a temperature of 60 °C was blown on the fiber or fibrous structure for a total of 4 minutes to solidify the material. The wet-processed fibrous dosage forms were square disks: 10 mm in side length and about 4 mm in thickness.

FIG. **17** shows a wet-processed single fiber **1710** exiting an exit port **1715.** Also shown is an exit port **1725** and stage **1730** while building a wet-processed fibrous dosage form **1720.**

For preparing wet-processed minimally-porous solid dosage forms, a stainless steel mold was placed on the top surface of the substrate and was filled with the fibrous stream exiting the exit port until a height of about 5 mm was reached. The material was left in the mold for 48 hours in a dry environment at 25°C to remove the solvent. Subsequently, the dosage form was ejected from the mold. The wet-processed minimally-porous dosage forms were circular disks: 13 mm in diameter and about 4 mm in thickness.

### Example 5: Drying of wet-processed single fibers

For determining the drying time of the wet fibers, a wet, single fiber was prepared as detailed above and deposited on a weighing boat. The fiber was then exposed to a stream of air at a temperature of 60 °C and a velocity of 2.3 m/s. The weight of the fiber was measured at the times 0, 50, 100, 150, 200, and 300 seconds after exposure to the air stream. The fraction of solvent (e.g., water) removed from the fiber, *m*(*t*)/*M*₀. was calculated as: $\frac{m \left(t\right)}{{M}_{0}} = 1 - \frac{w \left(t\right) - {w}_{∞}}{{w}_{0} - {w}_{∞}}$ (26) where *m*(*t*) is the weight of solvent removed from the fiber, *M*₀ the initial weight of solvent in the fiber, *w*(*t*) is the weigth of the fiber as a function of time, *w*₀ the initial weight of the plasticized (or wet) fiber, and *w*_{∞} the weight of the dry fiber.

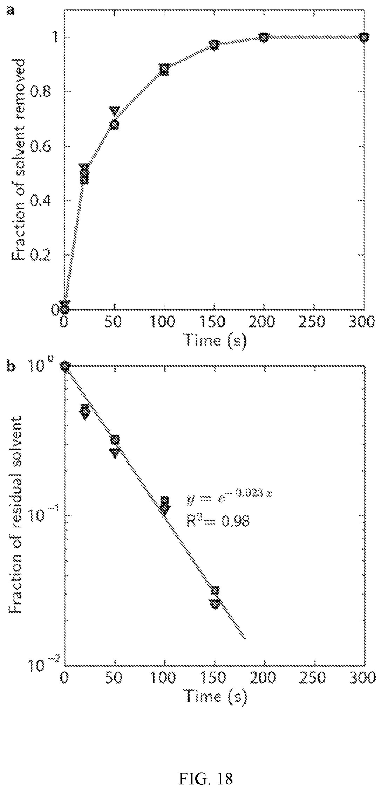

The fraction of solvent removed versus time after exposure of a wet fiber to the air stream (with *T* = 60 °C and *ν*_{*g,*∞} = 2.3 m/s) is shown in **FIG. 18a****.** *m*(*t*)/*M*₀ increases rapidly with time initially and then plateaus out to the final value of 1. **FIG. 18b** presents the fraction of residual solvent, 1-*m*(*t*)*M*₀, versus time. 1-*m*(*t*)/*M*₀ decays exponentially with time according to 1-*m*(*t*)/*M*₀ = 0.926×exp(-0.023*t*). Thus the drying time constant, *τ_{d} =* 1/0.023 = 43.48 s. The effective diffusivity of solvent in the fiber may be estimated directly from this result and Eqs. (16) and (17). Because *κ* >> 1, the first and dominant root of Eq. (17), *β*₁ = 2.4. Thus from Eq. (16), *D* ≈ *R_{f}*²/*τ_{d}β*₁² = 2.5×10⁻¹⁰ m²/s using *R_{f}* = 250 µm.

### Example 6: Estimation of the time during which fiber-to-fiber contact is deformable

The shear stress in contacting circular fibers is maximal at a distance *δ_{z}* ≈ 0.5*a* underneath the contact (*see, e.g.,* K.L. Johnson, "Contact mechanics", Cambridge University Press, 1985). Thus if the time during which the fiber-to-fiber contact is deformable, *τ_{def},* is approximated by the time the solvent molecules require to travel this distance, ${τ}_{def} \approx \frac{{δ}_{z}^{2}}{D} ≅ \frac{{a}^{2}}{4 D}$ (27) where *D* is the diffusivity of the solvent molecules in the fiber (as derived in section 4.1). Thus using *a* ≈ *R_{f}*/2 and *D* = 2.5×10⁻¹⁰ m²/s, *τ_{def}*≈ 15.6 seconds.

### Example 7: Microstructures of single fibers and dosage forms

For imaging the microstructure of the single fibers and dosage forms, the fiber or dosage form was first cut with a thin blade (MX35 Ultra, Thermo Scientific, Waltham, MA) to obtain a cross-section. The cross-section was then imaged using a Zeiss Merlin High Resolution SEM with a GEMINI column. Imaging was done with an in-lens secondary electron detector. An accelerating voltage of 5 kV and a probe current of 95 pA were applied to operate the microscope.

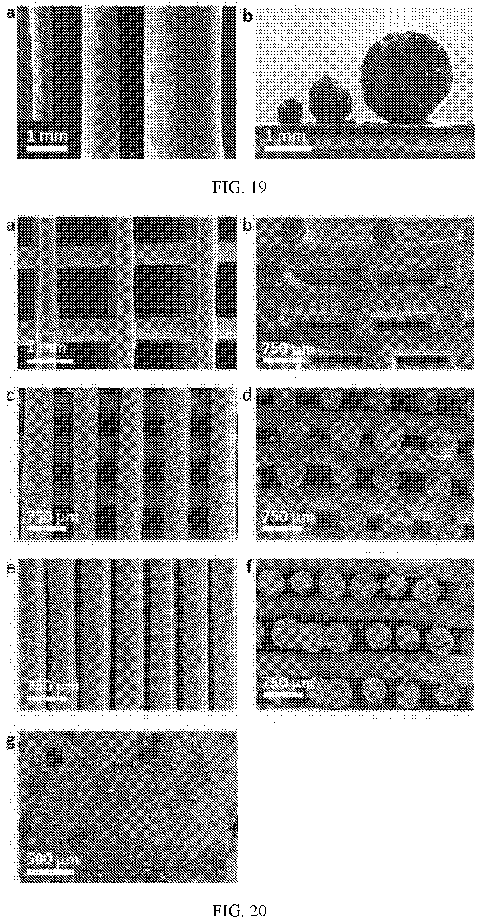

FIG. **19** presents representative SEM images of the melt-processed single fibers with drug particles embedded in an excipient matrix. The longitudinal views are given in FIG. **19a** and the FIG. **19b** shows the cross sections. The radii of the fibers are about 240, 456, and 954 µm, respectively, roughly the same as the inner diameter of the respective exit ports (Table 1).

Representative SEM images of the structures of melt-processed dosage forms are shown in FIG. **20.** FIGS. **20a****-f** are microstructures of the fibrous dosage forms. The initial fiber radius, *R*₀, and inter-fiber distance, *λ*₀, are predictable and agree well with the nominal parameters set by the *x-y-z* stage as summarized in Table 1. FIG. **20g** is the microstructure of an essentially non-porous solid dosage form with drug particles embedded in an excipient matrix.

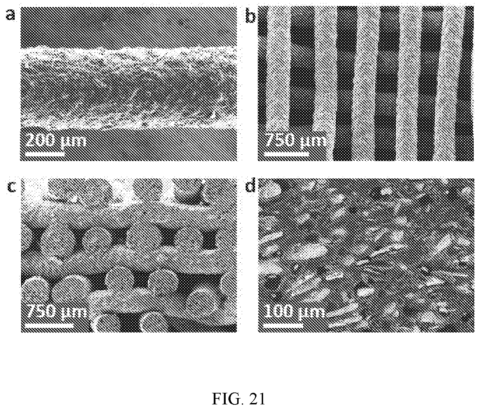

SEM images of a wet-processed single fiber, a wet-processed fibrous dosage form, and a wet-processed solid dosage form are shown in **FIG. 21.** FIG. **21a** is the longitudinal view of a single fiber with drug particles embedded in the excipient matrix. The diameter of the fiber is roughly 408 µm, slightly smaller than the inner diameter of the nozzle at the extruder exit. FIGS. **21b** and **21c** are the top and front views of the microstructure of the fibrous dosage form. The radius of the solid fibers, *Rₛ*, and the inter-fiber distance between the solid fibers, *λₛ*, are predictable by Eqs. (25a) and (25b) (replacing *R_{f}* and *λ_{f}* with the nominal parameters set by the radius of the exit port and the path of the *x-y-z* stage, as shown in Table 2). The semi-width of the crossed fibers contact was 172.3±23.4 µm, about the same as the value calculated by Eq. (12). Finally, FIG. **21d** is the cross section of a minimally-porous solid dosage form (the same as the cross section of a single fiber) with drug particles embedded in the excipient matrix.

### Example 8: Drug release of melt-processed single fibers and dosage forms

Drug release of the melt-processed single fibers and dosage forms was tested with a USP apparatus 1 (as shown in The United States Pharmacopeial Convention, USP 39-NF 34). The dosage forms were put in the dissolution basket inside a vessel filled with 900 ml of the dissolution fluid (a 0.05 M phosphate buffer solution prepared with sodium phosphate monobasic and sodium phosphate dibasic at a pH of 5.8 and at 37 °C). The basket was then rotated at 50 rpm. The concentration of dissolved drug was measured versus time by UV absorption at 240 nm using a fiber optic probe (Pion, Inc.).

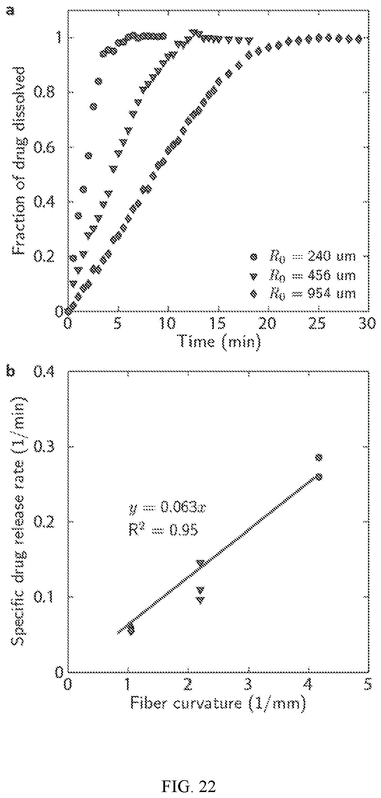

FIG. **22a** presents representative curves of the fraction of drug dissolved versus time by the melt-processed single fibers. For all the fibers, the fraction of drug dissolved increased steadily until it plateaued out to the final value. The times to dissolve 80% of the drug content, *t*_{0.8}, of the fibers tested are listed in Table 1. *t*_{0.8} increased as the initial fiber radius was increased, from about 2.9 minutes if *R*₀ = 240 µm to 14.25 minutes if *R*₀ = 954 µm. The drug release rate was limited by the rate at which the fibers eroded (e.g., by the erosion rate or disintegration rate of the fibers).

The approximate initial drug release flux from the fibers, ${j}_{d , 0} ≅ \frac{0.8 {M}_{d , 0}}{{t}_{0.8}} \frac{1}{{A}_{0}} = \frac{0.8}{{t}_{0.8}} \frac{{f}_{d} {ρ}_{s} {R}_{0}}{2}$ (28) is tabulated in Table 1 (0.8*M*_{*d*,0}/*t*_{0.8} is the time-average drug release rate, *A*₀ the initial fiber surface area, *f_{d}* the drug weight fraction in the fiber, and *ρₛ* the fiber density). *j*_{*d,*0} was between 0.21 and 0.26 µg/mm²s, roughly the same for all the fibers (Table 1). The dependence of the drug release flux on fiber radius was thus small.

The specific drug release rate (i.e., the time-average drug release rate divided by the initial drug content, 0.8/*t*_{0.8}) is plotted versus the fiber curvature (i.e., the inverse of the initial fiber radius, 1/*R*₀) in FIG. 22b. 0.8/*t*_{0.8} was roughly proportional to 1/*R*₀ and could be fitted to the curve 0.8/*t*_{0.8} = 0.063/*R*₀. Thus because the initial specific surface area of a fiber, *A*_{*s*,0} = 2/*R*₀, the specific drug release rate was directly proportional to *A*_{*s*,0}, 0.8/*t*_{0.8} = 0.033×*A*_{*s*,0}.

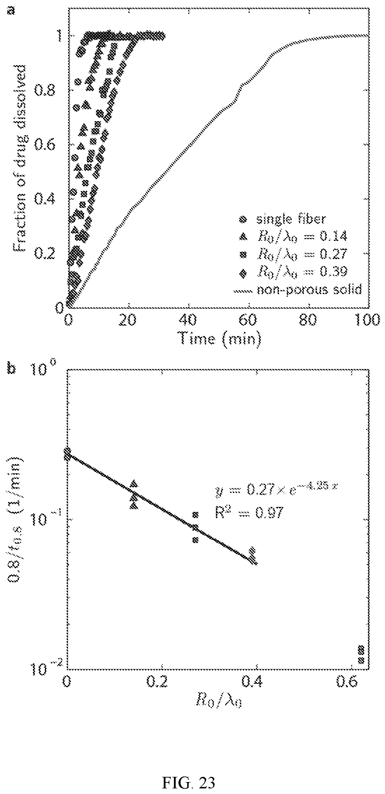

FIG. **23a** shows representative curves of the fraction of drug dissolved versus time of the fibrous dosage forms together with the data of a single fiber and the drug release results of the non-porous solid structure. *t*_{0.8} of the fibrous dosage forms was between 5.64 mins for the dosage form with *R*₀/*λ*₀ = 0.14, and 14.2 mins for *R*₀/*λ*₀ = 0.39, much faster than the drug release time of the corresponding non-porous solid dosage form with *t*_{0.8} = 63 mins.

FIG. **23b** presents the specific drug release rates of the dosage forms versus *R*₀/*λ*₀. The data of the fibrous forms could be fitted to an exponential curve. The specific release rates of the minimally-porous solid forms, however, did not follow this curve and were substantially smaller than "predicted" by the fit equation.

### Example 9: Drug release of wet-processed single fibers and dosage forms

Drug release of the wet-processed single fibers and dosage forms was tested with a USP apparatus 1 (as shown in The United States Pharmacopeial Convention, USP 39-NF 34), too. The dosage forms were put in the dissolution basket inside a vessel filled with 900 ml of the dissolution fluid (here a 0.05 M phosphate buffer solution prepared with sodium phosphate monobasic and sodium phosphate dibasic at a pH of 7.2 and at 37 °C). The basket was then rotated at 50 rpm. The concentration of dissolved drug was measured versus time by UV absorption at 220 nm using a fiber optic probe (Pion, Inc.).

FIG. **24** shows representative curves of the fraction of drug dissolved versus time of the wet-processed fibrous dosage forms together with the data of a wet-processed single fiber and the drug release results of the wet-processed minimally-porous solid dosage form. For all the fibers and dosage forms, the fraction of drug dissolved increased steadily with time until it plateaued out to the final value.

The average *t*_{0.8} values of the wet-processed dosage forms tested are listed in Table 2. The average *t*_{0.8} of the single fiber was roughly 3 mins. *t*_{0.8} increased if the fibers were assembled to a fibrous dosage form, to 7.8 mins. *t*_{0.8} of the fibrous dosage forms, however, was much smaller than the drug release time of the corresponding minimally-porous solid dosage form with *t*_{0.8} = 79.3 mins.

**Table 1.**

| Summary of microstructural parameters and drug dissolution times of melt-processed single fibers, melt-processed fibrous dosage forms, and melt-processed minimally-porous solid dosage forms. | | | | | | |
|---|---|---|---|---|---|---|
| | *R*₀ (µm) | *λ*₀ (µm) | *R*₀/*λ*₀ | *φₛ* | *t*_{0.8} (min) | *j*_{*d,*0} (µg/mm²s) |
| Single fibers | | | | | | |
| A | 240±6 | - | - | 0.0⁺ | 2.89 | 0.26 |
| B | 456±13 | - | - | 0.0⁺ | 7.03 | 0.21 |
| C | 954±16 | - | - | 0.0⁺ | 14.25 | 0.21 |

| Fibrous forms | | | | | | |
|---|---|---|---|---|---|---|
| D | 245±28 | 1783±47 | 0.14 | 0.22 | 5.64 | - |
| E | 253±17 | 922±38 | 0.27 | 0.43 | 9.14 | - |
| F | 243±13 | 629±70 | 0.39 | 0.61 | 14.17 | - |

| Minimally-porous solid form | | | | | | |
|---|---|---|---|---|---|---|
| G | - | - | - | 0.98 | 63.00 | 0.25 |

| | | | | | | |
|---|---|---|---|---|---|---|
| The nominal fiber radii, *Rₙ*, were 250 µm (A, D, E, and F), 500 µm (B), and 1000 µm (C). The nominal initial inter-fiber distances, *λₙ*, were 1750 µm (D), 900 µm (E), and 600 µm (F). The initial fiber radius, *R*₀, and fiber-to-fiber distance, *λ*₀, were obtained from the SEM images shown in Figs. 4 and 5. *t*_{0.8} is the time to dissolve 80% of the drug contained in the dosage form. It was derived from the results of drug release experiments shown in Figs. 8a and 9a. According to Eq. (1a) *φₛ* = *πR*₀/2*λ*₀. in the fibrous dosage forms. The initial drug release flux, *j*_{*d,*0}, of the single fibers was derived from Eq. (27) using *f_{d}* = 0.4, *t*_{0,8} as tabulated above, *ρₛ = f_{d}* × *ρ_{d}* + (1- *f_{d}*) × *ρₑ, ρ_{d}* = 1260 kg/m³, and *ρₑ* = 1150 kg/m³. The equation used to calculate the initial drug release flux from the faces of the minimally-porous solid dosage forms is: *j*_{*d*,0} = 0.8*H*₀*ρₛf_{d}*/2*t*_{0.8}*.* | | | | | | |

**Table 2.**

| Summary of microstructural parameters and drug dissolution times of wet-processed single fibers, wet-processed fibrous dosage forms, and wet-processed minimally-porous dosage forms. The nominal fiber diameter, 2*Rₙ* = 500 µm and the nominal inter-fiber distance, *λₙ* = 900 µm. | | | | | | |
|---|---|---|---|---|---|---|
| | 2*Rₛ* (µm) | *λₛ* (µm) | 2*aₛ* (µm) | *Rₛ*/*λₛ* | *φₛ* | to 8 (min) |
| **A** | 408±11 | - | - | - | - | 3.5 |
| **B** | 404±68 | 745±76 | 345±47 | 0.27 | 0.43 | 7.8 |
| **C** | - | - | - | - | - | 79.3 |

| | | | | | | |
|---|---|---|---|---|---|---|
| A: single fiber; B: fibrous dosage form; C: minimally-porous dosage form The volume fraction of the solid fibers, *φₛ* = *πRₛ*/2*λₛ.* The calculated values of *Rₛ* and *λₛ* are *Rₛ* ≥ 210 µm and *λₛ* ≥ 756 µm (replacing *R_{f}* and *λ_{f}* with *Rₙ* and *λₙ* in Eqs. (25a) and (25b)). The *t*_{0.8} values are an average of 3 samples. The *t*_{0.8} values of the individual samples were: 3, 3.5, and 4 min (single fiber), 7, 7.5, and 9 min (fibrous dosage form), and 77, 79, and 82 min (non-porous dosage form). | | | | | | |

### Example 10: Rheology of wet plasticized material

The shear viscosity of water-penetrated (e.g., plasticized) excipient-drug composite material was determined by mixing 40 wt% water with 36 wt% ibuprofen particles and 24 wt% Kollicoat IR. A shear rheometer (TA Instruments, ARG2 Rheometer, stress-controlled) equipped with a 60 mm diameter cone with an apex angle of 178° was used. The temperature was 37 °C during the experiments and the shear strain-rate range was 0.01-100 1/s.

FIG. **25** presents the results of the shear viscosity versus shear rate of the excipient-water-drug suspension with 40 wt% water, 36 wt% drug, and 24 wt% excipient (the composition of the plasticized or wet fibers during micro-patterning in the non-limiting experimental example 4). The shear viscosity of this formulation can be fitted to the curve ${μ}_{s} = 321 \times {\dot{γ}}_{s}^{- 0.65}$. Thus combining this result with Eq. (7) it follows that *m* = 321 and *n* = 0.35 in the non-limiting experimental example presented.

The shear viscosity of water-penetrated (e.g. plasticized) excipient alone was determined by first mixing water with Kollicoat IR at polymer weight fractions of 2.5, 5, 10, 15, 20, 25, 30, 35, and 40 wt% (the water weight fractions were 97.5, 95, 90, 85, 80, 75, 70, 65, and 60 wt%). The same equipment as above was used to execute the experiments. The temperature was 37 °C during the experiments, and the shear strain-rate range was 1-100 1/s.

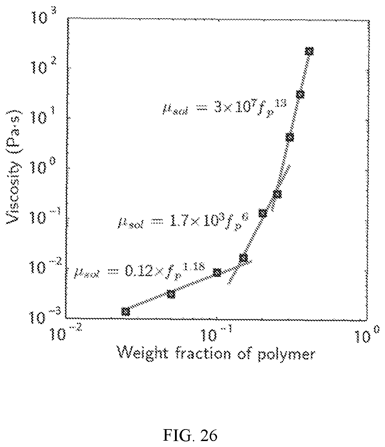

FIG. **26** presents the shear viscosity of excipient-water solutions (without any drug particles) versus weight fraction of polymer, *fₚ,* at the shear rate 1 1/s (*fₚ =* 1-*f_{w},* the weight fraction of water). At small weight fractions of the polymer (i.e. in the range 0.025 < *fₚ* < 0.14), the shear viscosity of the solution, *µₛₒₗ,* follows roughly *µₛₒₗ =* 0.12×*fₚ*^{1.18}. Then if 0.14 *< fₚ* < 0.25, the shear viscosity of the solution is about *µₛₒₗ* = 1.7×10³*fₚ*⁶, a much stronger dependence on *fₚ.* As the weight fraction of polymer is increased beyond 0.25, the curve of *µₛₒₗ* versus *fₚ* changes to an even stronger dependence on *fₚ.* In the range 0.25 < *fₚ* < 0.4 the viscosity of the solution roughly follows *µₛₒₗ* = 3×10⁷*fₚ*¹³. These results suggest that the solution is dilute up to *fₚ* ≈ 0.14, semidilute if 0.14 < *fₚ* < 0.25 and concentrated if *fₚ* is greater than about 0.25. Thus the polymer disentanglement concentration, *cₚ** ≈ 140 kg/m³, and the polymer concentration at the transition from a semi-dilute to a concentrated solution, *cₚ*** ≈ 250 kg/m³.

### Example 11: Shear strain rate in wet material during flow through exit port

During flow through the exit port of the extrusion channel, the shear strain rate in the wet, plasticized material is about: ${\dot{γ}}_{s , n} \approx \frac{Q}{{πR}_{n}^{3}}$ (29) Thus for *Q* = 2.83 mm³/s and *Rₙ* = 250 µm, *γ̇_{s,n}* ≈ 58 1/s. The shear viscosity of the wet material is about 40 Pa·s at this shear strain rate as shown in FIG. **25****.**

### ADDITIONAL APPLICATION EXAMPLES

The applicability of the apparatus and method herein is not limited to the processing of pharmaceutical materials (e.g., drugs and excipients) and the manufacture of fibrous dosage forms. It may also be applied for the manufacture of fibrous structures more generally. In the context of the invention herein, a fibrous structure is referred to a solid structure that is prepared from at least one fiber. This includes, but is not limited to solid structures that are prepared from at least one building block of one or more fibers (e.g., at least one fibrous building block). Thus, a fibrous structure may comprise a solid having an outer surface and an internal structure contiguous with and terminating at said outer surface. The internal structure may comprise a three dimensional structural network of one or more fibers. The fibers may further comprise fiber segments separated and spaced from adjoining fiber segments by free spacings, *λ_{f}*, which define one or more free spaces in the solid. The solid is not limited to a drug-containing solid. It may comprise an organic material, such as a food or food-like material (e.g., starch (e.g., potato starch, rice starch, corn starch, pregelatinized starch, etc.), amylose, amylopectin, polysaccharides, chocolate liquor, cocoa butter, cocoa, cocoa paste, fat, carbohydrates, lipids, proteins, vitamins, sweeteners (e.g., sugars or polyols (e.g. glucose, sucrose, mannitol, maltitol, sorbitol, maltodextrin, xylitol, etc.)), etc.), a polymer (e.g., polyethylene, polypropylene, polystyrene, polycarbonate. acrylonitrile butadiene styrene, etc.), a protein (e.g., collagen, glutelin, etc.), or an inorganic material, such as a metal (e.g., iron, aluminum, steel, stainless steel, copper, iridium, platinum, tungsten, etc.) or a ceramic.

### CONCLUDING REMARKS

In conclusion, this invention discloses methods and apparatuses for the manufacture of solid dosage forms with tailor-made microstructure. The manufacturing processes disclosed herein are further predictable, highly economical, and affords short process times. Thus the methods and apparatuses of this invention enable faster and more economical development and manufacture of high-quality and tailor-made pharmaceutical solid dosage forms or fibrous structures more generally.

It is contemplated that a particular feature described either individually or as part of an embodiment in this disclosure can be combined with other individually described features, or parts of other embodiments, even if the other features and embodiments make no mention of the particular feature without departing from the scope defined by the appended claims. Furthermore, the drawings and embodiments of the invention herein have been presented as examples, and not as limitations. Thus, it is to be understood that the invention herein is not limited to these precise embodiments. Other embodiments apparent to those of ordinary skill in the art are within the scope of what is claimed.

## Claims

1. (Currently amended) An apparatus (300) for the manufacture of pharmaceutical solid dosage forms comprising:
An internally hollow housing (340) having an internal surface encapsulating and defining an extrusion channel (330) having a first end (345) and a second end (350) and a cross section extending axially along its length from said first end (345) to said second end (350) and terminating into an exit port (350) at the second end (350);
said housing (340) having at least a first feeding port (355) between the first end (345) and second end (350) for injecting at least one solid constituent into the extrusion channel (330), and at least a second feeding port (356) between the first feeding port (355) and the exit port (350) for injecting at least one solvent into the extrusion channel (330) to form a plasticized matrix by solvating at least one injected solid constituent;
said apparatus (300) further comprising at least one conveying element (370) for extruding plasticized matrix in the extrusion channel (330) through an exit port (350) to form at least one plasticized fiber (360); and
a fiber structuring unit comprising a movable stage (380) for structuring one or more extruded plasticized fibers (360) to a three dimensional structural network by depositing said one or more fibers (360) along a path defined by motion of said stage (380);
wherein said stage on which said one or more fibers are deposited comprises a grid defining a deposition surface.

2. (Currently amended) A method of manufacturing pharmaceutical solid dosage forms comprising the steps of:
injecting one or more solid constituents (310, 320) into an extrusion channel (330) having a cross section extending along its length inside a housing (340);
injecting at least one solvent into said extrusion channel (330) to solvate at least one injected solid constituent (310, 320) so that the one or more injected solid constituents (310, 320) form a plasticized matrix;
conveying plasticized matrix towards an exit port (350) of the extrusion channel (330) by applying mechanical work on the plasticized matrix;
extruding plasticized matrix through an exit port (350) to form at least one plasticized fiber (360);
structuring one or more plasticized fibers (360) to a three-dimensional structural network by depositing said one or more fibers (360) along a path defined by motion of a movable stage (380); and
solidifying the deposited three dimensional structural network of one or more fibers;
whereby
the deposited three dimensional structural network of one or more fibers is solidified by a gas flow through the fibrous structure due to a pressure gradient of the gas across the fibrous structure.

3. (Currently amended) An apparatus (400) for the manufacture of pharmaceutical solid dosage forms comprising:
An internally hollow housing (440) having an internal surface encapsulating and defining an extrusion channel (430) having a first end (445) and a second end (450) and a cross section extending axially along its length from said first end (445) to said second end (450) and terminating into an exit port (450) at the second end (450);
said housing (440) having at least a first feeding port (455) between the first end (445) and second end (450) for injecting at least one solid constituent into the extrusion channel (430);
said apparatus (400) further comprising at least one heating element (486) for fluidizing at least one injected solid constituent so that the injected one or more solid constituents form a plasticized matrix in the extrusion channel (430);
at least one conveying element (470) for extruding plasticized matrix in the extrusion channel (430) through an exit port (450) to form at least one plasticized fiber (460); and
a fiber structuring unit comprising a movable stage (480) for structuring one or more extruded plasticized fibers (460) to a three dimensional structural network by depositing said one or more fibers (460) along a path defined by motion of said stage (480);
wherein said stage on which said one or more fibers are deposited comprises a grid defining a deposition surface.

4. (Currently amended) A method of manufacturing pharmaceutical solid dosage forms comprising the steps of:
injecting one or more solid constituents (410, 420) into an extrusion channel (430) having a cross section extending along its length inside a housing (440), wherein at least one solid constituent (410, 420) melts upon heating;
heating the injected one or more solid constituents (410, 420) to form a plasticized matrix;
conveying plasticized matrix towards an exit port (450) of the extrusion channel (430) by applying mechanical work on the plasticized matrix;
extruding plasticized matrix through an exit port (450) to form at least one plasticized fiber (460);
structuring one or more plasticized fibers (460) to a three-dimensional structural network by depositing said one or more fibers (460) along a path defined by motion of a movable stage (480); and
solidifying the deposited three dimensional structural network of one or more fibers;
whereby
the deposited three dimensional structural network of one or more fibers is solidified by a gas flow through the fibrous structure due to a pressure gradient of the gas across the fibrous structure.

5. (Currently amended) An apparatus (500) for the manufacture of pharmaceutical solid dosage forms comprising:
An internally hollow housing (540) having an internal surface encapsulating and defining an extrusion channel (530) having a first end (545) and a second end (550) and a cross section extending axially along its length from said first end (545) to said second end (550) and terminating into an exit port (550) at the second end (550);
said housing (540) having at least a feeding port (555) between the first end (545) and second end (550) for injecting a plasticized matrix into the extrusion channel (530);
said apparatus further comprising at least one conveying element (570) for extruding plasticized matrix in the extrusion channel (530) through an exit port (550) to form at least one plasticized fiber (560); and
a fiber structuring unit comprising a movable stage (580) for structuring one or more extruded fibers (560) to a three dimensional structural network by depositing said one or more fibers (560) along a path defined by motion of said stage (580);
wherein said stage on which said one or more fibers are deposited comprises a grid defining a deposition surface.

6. (Currently amended) A method of manufacturing pharmaceutical solid dosage forms comprising the steps of:
injecting a plasticized matrix into an extrusion channel (530) having a cross section extending along its length inside a housing (540);
conveying plasticized matrix towards an exit port (550) of the extrusion channel (530) by applying mechanical work on the plasticized matrix;
extruding plasticized matrix through an exit port (550) to form at least one plasticized fiber (560);
structuring one or more extruded plasticized fibers (560) to a three-dimensional structural network by depositing said one or more fibers (560) along a path defined by motion of a movable stage (580); and
solidifying the deposited three dimensional structural network of one or more fibers;
whereby
the deposited three dimensional structural network of one or more fibers is solidified by a gas flow through the fibrous structure due to a pressure gradient of the gas across the fibrous structure.

7. (Previously presented) The apparatus of any preceding claims, wherein the extrusion channel (330, 430, 530) cross section tapers down before an exit port (350, 450, 550) to the cross section of said exit port (350, 450, 550); and/or the apparatus of any preceding claims, wherein the extrusion channel (330, 430, 530) bifurcates into at least one other end comprising an exit port; and/or the apparatus of any preceding claims, further comprising at least one solvent feeding unit (386) attached to at least the second feeding port (356) for injecting at least one solvent into the extrusion channel (330); and/or the apparatus of any preceding claims, further comprising a granular solids feeding unit (385, 485) for injecting one or more granular solid constituents through the first feeding port (355, 455) into the extrusion channel (330, 430).

8. (Previously presented) The method of claim 2, wherein injecting at least one solvent into the extrusion channel (330) is performed using a solvent feeding unit (386) attached to at least a second feeding port (356) of the extrusion channel (330).

9. (Previously presented) The method of any preceding claims, wherein at least one of the one or more injected solid constituents (310, 320, 410, 420) is a granular solid constituent, and wherein said granular solid constituent is injected into the extrusion channel (330, 430) using a granular solids feeding unit (385, 485).

10. (Previously presented) The method of any preceding claims, wherein conveying the plasticized matrix towards an exit port (350, 450, 550) of the extrusion channel (330, 430, 530) is performed using a conveying element (370, 470, 570) selected from the group comprising screws or pistons.

11. (Previously presented) The method of any preceding claims, wherein the stage comprises an *x-y-z* stage; and/or the method of any preceding claims, wherein the stage comprises a grid defining a deposition surface; and/or the method of any preceding claims, wherein the stage comprises a grid defining a deposition surface that is impermeable to a deposited fiber but permits air or gas flow through it; and/or the method of any preceding claims, wherein the stage comprises a grid through which gas flows for solidifying a deposited three dimensional structural network of fibers; and/or the method of any preceding claims, wherein the stage comprises a grid through which gas flows for removing solvent from a deposited three dimensional structural network of fibers; and/or the method of any preceding claims, wherein the stage comprises a grid through which gas flows for promoting solidification of a deposited three dimensional structural network of fibers by evaporating solvent or by cooling.

12. (Previously presented) The apparatus of any preceding claims, wherein the stage comprises an *x-y-z* stage; and/or the apparatus of any preceding claims, wherein the grid defines a deposition surface that is impermeable to a deposited fiber but permits air or gas flow through it; and/or the apparatus of any preceding claims, wherein gas can flow through the grid for solidifying a deposited three dimensional structural network of fibers; and/or the apparatus of any preceding claims, wherein gas can flow through the grid for removing solvent from a deposited three dimensional structural network of fibers; and/or the apparatus of any preceding claims, wherein gas can flow through the grid for promoting solidification of a deposited three dimensional structural network of fibers by evaporating solvent or by cooling.

13. (Previously presented) The apparatus of any preceding claims, wherein at least one conveying element (370, 470, 570) is selected from the group comprising screws or pistons; and/or the apparatus of any preceding claims, further comprising a unit for blowing gas on a deposited structure; and/or the apparatus of any preceding claims, wherein at least one exit port (350, 450, 550) is designed to extrude fiber having an average fiber thickness less than 2.5 mm.

14. (Previously presented) The method of any preceding claims, further comprising a unit for blowing gas on a deposited structure.

15. (Previously presented) The method of any preceding claims, wherein at least one exit port (350, 450, 550) is designed to extrude fiber having an average fiber thickness less than 2.5 mm.

16. (Previously presented) The method of any preceding claims, wherein the solid, three dimensional structural network of fibers (110, 120, 130, 140, 150, 160, 170, 180) comprises at least one inter-fiber point contact, said inter-fiber point contact having a contact width no greater than 2 mm; and/or the method of any preceding claims, wherein the solid, three dimensional structural network of fibers (110, 120, 130, 140, 150, 160, 170, 180) comprises at least one inter-fiber point contact, and wherein the number of said inter-fiber point contacts in the three dimensional structural network of fibers (110, 120, 130, 140, 150, 160, 170, 180) is precisely controlled.

17. (Previously presented) The method of any preceding claims, wherein the thickness of a fiber and an inter-fiber spacing in the solid, three dimensional structural network of fibers (110, 120, 130, 140, 150, 160, 170, 180) are precisely controlled; and/or the method of any preceding claims wherein the three dimensional structural network of fibers (110, 120, 130, 140, 150, 160, 170, 180) comprises an ordered or partially ordered structure; and/or the method of any preceding claims wherein the pharmaceutical solid dosage form comprises a drug-containing solid (101) having an outer surface (102) and an internal structure (104) contiguous with and terminating at said outer surface (102), said internal structure (104) comprising a three dimensional structural network of one or more fibers (110, 120, 130, 140, 150, 160, 170, 180), said fibers (110, 120, 130, 140, 150, 160, 170, 180) comprising at least one active ingredient and at least one excipient, said fibers (110, 120, 130, 140, 150, 160, 170, 180) further comprising fiber segments separated and spaced from adjoining fiber segments by free spacings, and the free spacings defining one or more free spaces (105) in a drug-containing solid (101), whereby the one or more fibers (110, 120, 130, 140, 150, 160, 170, 180) comprise an average thickness between 1 µm and 2.5 mm, and the effective free spacing between fiber segments across the one or more free spaces (105) on average is greater than 1 µm.

## Patentansprüche

1. Eine Vorrichtung (300) zur Herstellung pharmazeutischer fester Darreichungsformen, umfassend:
ein innen hohles Gehäuse (340) mit einer Innenfläche, die einen Extrusionskanal (330) mit einem ersten Ende (345) und einem zweiten Ende (350) umschließt und definiert, sowie einem Querschnitt, der sich axial entlang seiner Länge vom ersten Ende (345) zum zweiten Ende (350) erstreckt und am zweiten Ende (350) in einer Auslassöffnung (350) endet;
das Gehäuse (340) weist mindestens eine erste Zufuhröffnung (355) zwischen dem ersten Ende (345) und dem zweiten Ende (350) zum Einbringen mindestens eines festen Bestandteils in den Extrusionskanal (330) und mindestens eine zweite Zufuhröffnung (356) zwischen der ersten Zufuhröffnung (355) und der Auslassöffnung (350) zum Einbringen mindestens eines Lösungsmittels in den Extrusionskanal (330) auf, um durch Solvatisierung mindestens eines eingebrachten festen Bestandteils eine plastifizierte Matrix zu bilden;
Die Vorrichtung (300) umfasst ferner mindestens ein Förderelement (370) zum Extrudieren der plastifizierten Matrix im Extrusionskanal (330) durch eine Austrittsöffnung (350) zur Bildung mindestens einer plastifizierten Faser (360); und
eine Faserstrukturierungseinheit mit einem beweglichen Tisch (380) zum Strukturieren einer oder mehrerer der extrudierten plastifizierten Fasern (360) zu einem dreidimensionalen Strukturnetzwerk durch Ablagerung der Fasern (360) entlang eines durch die Bewegung des Tisches (380) definierten Pfads;
wobei der Tisch, auf dem die Fasern abgelagert werden, ein Gitter mit einer Ablagerungsfläche aufweist.

2. Ein Verfahren zur Herstellung pharmazeutischer fester Darreichungsformen, umfassend die folgenden Schritte:
Einführen eines oder mehrerer fester Bestandteile (310, 320) in einen Extrusionskanal (330), der innerhalb eines Gehäuses (340) verläuft und einen sich entlang seiner Länge erstreckenden Querschnitt aufweist;
Einführen mindestens eines Lösungsmittels in den Extrusionskanal (330), um mindestens einen der zugeführten festen Bestandteile (310, 320) zu lösen, sodass der eine oder die mehreren zugeführten festen Bestandteile (310, 320) eine plastifizierte Matrix bilden;
Fördern der plastifizierten Matrix in Richtung einer Austrittsöffnung (350) des Extrusionskanals (330) durch Ausüben mechanischer Arbeit auf die plastifizierte Matrix;
Extrudieren der plastifizierten Matrix durch eine Austrittsöffnung (350) zur Bildung mindestens einer plastifizierten Faser (360);
Strukturieren einer oder mehrerer plastifizierter Fasern (360) zu einem dreidimensionalen strukturellen Netzwerk durch Ablegen der einen oder mehreren Fasern (360) entlang eines Pfades, der durch die Bewegung einer beweglichen Plattform (380) definiert wird; und
Verfestigen des abgelegten dreidimensionalen strukturellen Netzwerks aus einer oder mehreren Fasern;
wobei
das abgelegte dreidimensionale strukturelle Netzwerk aus einer oder mehreren Fasern durch einen Gasfluss durch die Faserstruktur verfestigt wird, der durch einen Druckgradienten des Gases über der Faserstruktur entsteht.

3. Eine Vorrichtung (400) zur Herstellung pharmazeutischer fester Darreichungsformen, umfassend:
ein innen hohles Gehäuse (440) mit einer Innenfläche, die einen Extrusionskanal (430) umschließt und definiert, wobei der Extrusionskanal ein erstes Ende (445) und ein zweites Ende (450) aufweist, einen Querschnitt besitzt, der sich axial entlang seiner Länge von dem ersten Ende (445) bis zu dem zweiten Ende (450) erstreckt, und an dem zweiten Ende (450) in eine Austrittsöffnung (450) mündet;
wobei das Gehäuse (440) zwischen dem ersten Ende (445) und dem zweiten Ende (450) mindestens eine Zuführöffnung (455) zum Einbringen mindestens eines festen Bestandteils in den Extrusionskanal (430) aufweist;
wobei die Vorrichtung (400) ferner mindestens ein Heizelement (486) umfasst, um mindestens einen eingebrachten festen Bestandteil zu fluidisieren, sodass der eine oder die mehreren eingebrachten festen Bestandteile eine plastifizierte Matrix im Extrusionskanal (430) bilden;
mindestens ein Förderelement (470) zum Extrudieren der plastifizierten Matrix aus dem Extrusionskanal (430) durch eine Austrittsöffnung (450), um mindestens eine plastifizierte Faser (460) zu bilden; und
eine Faserstrukturierungseinheit, die eine bewegliche Plattform (480) umfasst, um eine oder mehrere extrudierte plastifizierte Fasern (460) zu einem dreidimensionalen strukturellen Netzwerk zu strukturieren, indem die eine oder die mehreren Fasern (460) entlang eines Pfades abgelegt werden, der durch die Bewegung der Plattform (480) definiert ist;
wobei die Plattform, auf der die eine oder die mehreren Fasern abgelegt werden, ein Gitter umfasst, das eine Ablagefläche definiert.

4. Verfahren zur Herstellung pharmazeutischer fester Darreichungsformen, umfassend die folgenden Schritte:
Einführen eines oder mehrerer fester Bestandteile (410, 420) in einen Extrusionskanal (430), der einen sich entlang seiner Länge erstreckenden Querschnitt aufweist und in einem Gehäuse (440) angeordnet ist, wobei mindestens ein fester Bestandteil (410, 420) beim Erhitzen schmilzt;
Erhitzen des einen oder der mehreren eingeführten festen Bestandteile (410, 420) zur Bildung einer plastifizierten Matrix;
Fördern der plastifizierten Matrix in Richtung einer Austrittsöffnung (450) des Extrusionskanals (430) durch Ausüben mechanischer Arbeit auf die plastifizierte Matrix;
Extrudieren der plastifizierten Matrix durch eine Austrittsöffnung (450) zur Bildung mindestens einer plastifizierten Faser (460);
Strukturieren einer oder mehrerer plastifizierter Fasern (460) zu einem dreidimensionalen strukturellen Netzwerk durch Ablegen der einen oder mehreren Fasern (460) entlang eines Pfades, der durch die Bewegung einer beweglichen Plattform (480) definiert wird; und
Verfestigen des abgelegten dreidimensionalen strukturellen Netzwerks aus einer oder mehreren Fasern;
wobei
das abgelegte dreidimensionale strukturelle Netzwerk aus einer oder mehreren Fasern durch einen Gasfluss durch die Faserstruktur verfestigt wird, der durch einen Druckgradienten des Gases über der Faserstruktur entsteht.

5. Eine Vorrichtung (500) zur Herstellung von festen pharmazeutischen Darreichungsformen, umfassend:
Ein innen hohles Gehäuse (540) mit einer Innenfläche, die einen Extrusionskanal (530) umschließt und definiert, wobei der Extrusionskanal ein erstes Ende (545) sowie ein zweites Ende (550) aufweist, einen Querschnitt besitzt, der sich axial entlang seiner Länge von dem ersten Ende (545) bis zu dem zweiten Ende (550) erstreckt, und an dem zweiten Ende (550) in eine Austrittsöffnung (550) mündet;
wobei das Gehäuse (540) zwischen dem ersten Ende (545) und dem zweiten Ende (550) mindestens eine Zuführöffnung (555) zum Einbringen einer plastifizierten Matrix in den Extrusionskanal (530) aufweist;
wobei die Vorrichtung ferner mindestens ein Förderelement (570) umfasst, um die plastifizierte Matrix im Extrusionskanal (530) durch eine Austrittsöffnung (550) zu extrudieren und so mindestens eine plastifizierte Faser (560) zu bilden; und
eine Faserstrukturierungseinheit, die eine bewegliche Plattform (480) umfasst, um eine oder mehrere extrudierte plastifizierte Fasern (460) zu einem dreidimensionalen strukturellen Netzwerk zu strukturieren, indem die eine oder die mehreren Fasern (460) entlang eines Pfades abgelegt werden, der durch die Bewegung der Plattform (480) definiert ist;
wobei die Plattform, auf der die eine oder die mehreren Fasern abgelegt werden, ein Gitter umfasst, das eine Ablagefläche definiert.

6. Verfahren zur Herstellung pharmazeutischer fester Darreichungsformen, umfassend die folgenden Schritte:
Einführen einer plastifizierten Matrix in einen Extrusionskanal (530), der innerhalb eines Gehäuses (540) einen sich entlang seiner Länge erstreckenden Querschnitt aufweist;
Fördern der plastifizierten Matrix in Richtung einer Austrittsöffnung (550) des Extrusionskanals (530) durch Ausüben mechanischer Arbeit auf die plastifizierte Matrix;
Extrudieren der plastifizierten Matrix durch eine Austrittsöffnung (550) zur Bildung mindestens einer plastifizierten Faser (560);
Strukturieren einer oder mehrerer extrudierter plastifizierter Fasern (560) zu einem dreidimensionalen strukturellen Netzwerk durch Ablegen der einen oder mehreren Fasern (560) entlang eines Pfades, der durch die Bewegung eines beweglichen Trägers (580) definiert wird; und
Verfestigen des abgelegten dreidimensionalen strukturellen Netzwerks aus einer oder mehreren Fasern;
wobei
das abgelegte dreidimensionale strukturelle Netzwerk aus einer oder mehreren Fasern durch einen Gasfluss durch die Faserstruktur verfestigt wird, der durch einen Druckgradienten des Gases über der Faserstruktur entsteht.

7. Vorrichtung nach einem der vorhergehenden Ansprüche, wobei sich der Querschnitt des Extrusionskanals (330, 430, 530) vor einer Austrittsöffnung (350, 450, 550) auf den Querschnitt dieser Austrittsöffnung (350, 450, 550) verjüngt; und/oder Vorrichtung nach einem der vorhergehenden Ansprüche, wobei sich der Extrusionskanal (330, 430, 530) in mindestens ein weiteres Ende aufteilt, das eine Austrittsöffnung umfasst; und/oder Vorrichtung nach einem der vorhergehenden Ansprüche, ferner umfassend mindestens eine Lösungsmittel-Zufuhreinheit (386), die mit zumindest der zweiten Zufuhröffnung (356) verbunden ist, um mindestens ein Lösungsmittel in den Extrusionskanal (330) einzuleiten; und/oder Vorrichtung nach einem der vorhergehenden Ansprüche, ferner umfassend eine Zufuhreinheit für körnige Feststoffe (385, 485) zum Einleiten eines oder mehrerer körniger Feststoffbestandteile durch die erste Zufuhröffnung (355, 455) in den Extrusionskanal (330, 430).

8. Verfahren nach Anspruch 2, wobei das Injizieren mindestens eines Lösungsmittels in den Extrusionskanal (330) mithilfe einer Lösungsmittelzufuhreinheit (386) erfolgt, die an mindestens einen zweiten Zufuhranschluss (356) des Extrusionskanals (330) angeschlossen ist.

9. Verfahren nach einem der vorhergehenden Ansprüche, wobei es sich bei mindestens einem der einen oder mehreren injizierten festen Bestandteile (310, 320, 410, 420) um einen körnigen festen Bestandteil handelt, und wobei dieser körnige feste Bestandteil mittels einer Zuführeinheit für körnige Feststoffe (385, 485) in den Extrusionskanal (330, 430) injiziert wird.

10. Verfahren nach einem der vorhergehenden Ansprüche, wobei das Fördern der plastifizierten Matrix in Richtung einer Austrittsöffnung (350, 450, 550) des Extrusionskanals (330, 430, 530) mittels eines Förderelements (370, 470, 570) erfolgt, das aus der Gruppe bestehend aus Schnecken oder Kolben ausgewählt ist.

11. Verfahren nach einem der vorhergehenden Ansprüche, wobei die Auflage einen X-Y-Z-Tisch umfasst; und/oder Verfahren nach einem der vorhergehenden Ansprüche, wobei die Auflage ein Gitter umfasst, das eine Ablagerungsfläche definiert; und/oder Verfahren nach einem der vorhergehenden Ansprüche, wobei die Auflage ein Gitter umfasst, das eine Ablagerungsfläche definiert, die für eine abgelagerte Faser undurchlässig ist, jedoch einen Luft- oder Gasstrom durch sie hindurch zulässt; und/oder Verfahren nach einem der vorhergehenden Ansprüche, wobei die Auflage ein Gitter umfasst, durch das Gas strömt, um ein abgelagertes dreidimensionales strukturelles Fasernetzwerk zu verfestigen; und/oder Verfahren nach einem der vorhergehenden Ansprüche, wobei die Auflage ein Gitter umfasst, durch das Gas strömt, um Lösungsmittel aus einem abgelagerten dreidimensionalen strukturellen Fasernetzwerk zu entfernen; und/oder Verfahren nach einem der vorhergehenden Ansprüche, wobei die Auflage ein Gitter umfasst, durch das Gas strömt, um die Verfestigung eines abgelagerten dreidimensionalen strukturellen Fasernetzwerks durch Verdampfen von Lösungsmittel oder durch Abkühlen zu fördern.

12. Vorrichtung nach einem der vorhergehenden Ansprüche, wobei der Auflagetisch einen X-Y-Z-Auflagetisch umfasst; und/oder Vorrichtung nach einem der vorhergehenden Ansprüche, wobei das Gitter eine Ablagefläche definiert, die für eine abgelagerte Faser undurchlässig ist, jedoch einen Luft- oder Gasstrom durch sie hindurch zulässt; und/oder Vorrichtung nach einem der vorhergehenden Ansprüche, wobei Gas durch das Gitter strömen kann, um ein abgelagertes dreidimensionales strukturelles Fasernetzwerk zu verfestigen; und/oder Vorrichtung nach einem der vorhergehenden Ansprüche, wobei Gas durch das Gitter strömen kann, um Lösungsmittel aus einem abgelagerten dreidimensionalen strukturellen Fasernetzwerk zu entfernen; und/oder Vorrichtung nach einem der vorhergehenden Ansprüche, wobei Gas durch das Gitter strömen kann, um die Verfestigung eines abgelagerten dreidimensionalen strukturellen Fasernetzwerks durch Verdampfen von Lösungsmittel oder durch Abkühlen zu fördern.

13. Vorrichtung nach einem der vorhergehenden Ansprüche, wobei mindestens ein Förderelement (370, 470, 570) aus der Gruppe bestehend aus Schnecken oder Kolben ausgewählt ist; und/oder Vorrichtung nach einem der vorhergehenden Ansprüche, ferner umfassend eine Einheit zum Beaufschlagen einer abgelagerten Struktur mit einem Gasstrom; und/oder Vorrichtung nach einem der vorhergehenden Ansprüche, wobei mindestens eine Austrittsöffnung (350, 450, 550) dazu ausgelegt ist, Fasern mit einer durchschnittlichen Faserdicke von weniger als 2,5 mm zu extrudieren.

14. Verfahren nach einem der vorhergehenden Ansprüche, ferner umfassend eine Einheit zum Blasen von Gas auf eine abgelegte Struktur.

15. Verfahren nach einem der vorhergehenden Ansprüche, wobei mindestens eine Austrittsöffnung (350, 450, 550) dazu ausgelegt ist, Fasern mit einer durchschnittlichen Faserdicke von weniger als 2,5 mm zu extrudieren.

16. Verfahren nach einem der vorhergehenden Ansprüche, wobei das feste, dreidimensionale strukturelle Netzwerk aus Fasern (110, 120, 130, 140, 150, 160, 170, 180) mindestens einen Punktkontakt zwischen Fasern umfasst, wobei dieser Punktkontakt zwischen Fasern eine Kontaktbreite von höchstens 2 mm aufweist; und/oder Verfahren nach einem der vorhergehenden Ansprüche, wobei das feste, dreidimensionale strukturelle Netzwerk aus Fasern (110, 120, 130, 140, 150, 160, 170, 180) mindestens einen Punktkontakt zwischen Fasern umfasst, und wobei die Anzahl dieser Punktkontakte zwischen Fasern in dem dreidimensionalen strukturellen Netzwerk aus Fasern (110, 120, 130, 140, 150, 160, 170, 180) präzise gesteuert wird.

17. Verfahren nach einem der vorhergehenden Ansprüche, wobei die Dicke einer Faser sowie der Abstand zwischen den Fasern in dem festen, dreidimensionalen strukturellen Netzwerk aus Fasern (110, 120, 130, 140, 150, 160, 170, 180) präzise gesteuert werden; und/oder Verfahren nach einem der vorhergehenden Ansprüche, wobei das dreidimensionale strukturelle Netzwerk aus Fasern (110, 120, 130, 140, 150, 160, 170, 180) eine geordnete oder teilweise geordnete Struktur aufweist; und/oder das Verfahren nach einem der vorstehenden Ansprüche, wobei die pharmazeutische feste Darreichungsform einen wirkstoffhaltigen Festkörper (101) umfasst, der eine Außenfläche (102) sowie eine innere Struktur (104) aufweist, die an die Außenfläche (102) angrenzt und an dieser endet; wobei die innere Struktur (104) ein dreidimensionales strukturelles Netzwerk aus einer oder mehreren Fasern (110, 120, 130, 140, 150, 160, 170, 180) umfasst, wobei diese Fasern (110, 120, 130, 140, 150, 160, 170, 180) mindestens einen Wirkstoff und mindestens einen Hilfsstoff enthalten; wobei die Fasern (110, 120, 130, 140, 150, 160, 170, 180) ferner Fasersegmente umfassen, die durch freie Zwischenräume von benachbarten Fasersegmenten getrennt und beabstandet sind, wobei diese freien Zwischenräume einen oder mehrere Hohlräume (105) innerhalb des wirkstoffhaltigen Festkörpers (101) definieren; und wobei die eine oder die mehreren Fasern (110, 120, 130, 140, 150, 160, 170, 180) eine durchschnittliche Dicke zwischen 1 µm und 2,5 mm aufweisen und der effektive freie Abstand zwischen Fasersegmenten über den einen oder die mehreren Hohlräume (105) hinweg im Durchschnitt größer als 1 µm ist.

## Revendications

1. Un appareil (300) destiné à la fabrication de formes galéniques solides pharmaceutiques, comprenant:
un boîtier (340) creux, doté d'une surface interne qui délimite un canal d'extrusion (330) ; ce canal présente une première extrémité (345), une seconde extrémité (350) et une section transversale s'étendant axialement sur toute sa longueur, depuis ladite première extrémité (345) jusqu'à ladite seconde extrémité (350), pour aboutir à un orifice de sortie (350) situé au niveau de la seconde extrémité (350);
ledit boîtier (340) comporte au moins un premier orifice d'alimentation (355), situé entre la première extrémité (345) et la seconde extrémité (350), destiné à l'injection d'au moins un constituant solide dans le canal d'extrusion (330), ainsi qu'au moins un second orifice d'alimentation (356), situé entre le premier orifice d'alimentation (355) et l'orifice de sortie (350), destiné à l'injection d'au moins un solvant dans le canal d'extrusion (330) afin de former une matrice plastifiée par solvatation d'au moins un constituant solide injecté;
ledit appareil (300) comprend en outre au moins un élément de transport (370) servant à extruder la matrice plastifiée présente dans le canal d'extrusion (330) à travers l'orifice de sortie (350), de manière à former au moins une fibre plastifiée (360); et
une unité de structuration de fibres, comprenant un plateau mobile (380) conçu pour structurer une ou plusieurs fibres plastifiées extrudées (360) en un réseau structurel tridimensionnel, par le dépôt de ladite ou desdites fibres (360) le long d'un trajet défini par le mouvement dudit plateau (380);
ledit plateau, sur lequel sont déposées la ou les fibres, comprenant une grille qui définit une surface de dépôt.

2. Procédé de fabrication de formes galéniques solides, comprenant les étapes consistant à:
introduire un ou plusieurs constituants solides (310, 320) dans un canal d'extrusion (330) présentant une section transversale qui s'étend sur toute sa longueur à l'intérieur d'un corps (340);
introduire au moins un solvant dans ledit canal d'extrusion (330) afin de solvater au moins un constituant solide introduit (310, 320), de sorte que le ou les constituants solides introduits (310, 320) forment une matrice plastifiée;
acheminer la matrice plastifiée vers un orifice de sortie (350) du canal d'extrusion (330) en exerçant une action mécanique sur ladite matrice;
extruder la matrice plastifiée à travers un orifice de sortie (350) pour former au moins une fibre plastifiée (360);
structurer une ou plusieurs fibres plastifiées (360) en un réseau structurel tridimensionnel, par dépôt de ladite ou desdites fibres (360) le long d'un trajet défini par le déplacement d'un support mobile (380); et
solidifier le réseau structurel tridimensionnel formé par la ou les fibres déposées;
le réseau structurel tridimensionnel formé par la ou les fibres déposées étant solidifié par un flux de gaz traversant la structure fibreuse, sous l'effet d'un gradient de pression du gaz à travers ladite structure.

3. Un appareil (400) destiné à la fabrication de formes galéniques pharmaceutiques solides, comprenant:
un boîtier (440) creux, doté d'une surface interne qui délimite un canal d'extrusion (430) ; ce canal présente une première extrémité (445), une seconde extrémité (450) et une section transversale s'étendant axialement sur toute sa longueur, depuis ladite première extrémité (445) jusqu'à ladite seconde extrémité (450), pour aboutir à un orifice de sortie (450) situé au niveau de la seconde extrémité (450);
ledit boîtier (440) comporte au moins un premier orifice d'alimentation (455), situé entre la première extrémité (445) et la seconde extrémité (450), permettant l'injection d'au moins un constituant solide dans le canal d'extrusion (430);
ledit appareil (400) comprend en outre au moins un élément chauffant (486) destiné à fluidifier au moins un constituant solide injecté, de sorte que le ou les constituants solides injectés forment une matrice plastifiée à l'intérieur du canal d'extrusion (430);
au moins un élément de transport (470) servant à extruder la matrice plastifiée présente dans le canal d'extrusion (430) à travers un orifice de sortie (450), afin de former au moins une fibre plastifiée (460); et
une unité de structuration de fibres comprenant un plateau mobile (480) destiné à structurer une ou plusieurs fibres plastifiées extrudées (460) en un réseau structurel tridimensionnel, par le dépôt de ladite ou desdites fibres (460) le long d'un trajet défini par le mouvement dudit plateau (480);
ledit plateau, sur lequel sont déposées la ou les fibres, comprenant une grille qui définit une surface de dépôt.

4. Procédé de fabrication de formes galéniques solides, comprenant les étapes suivantes:
l'introduction d'un ou plusieurs constituants solides (410, 420) dans un canal d'extrusion (430) présentant une section transversale qui s'étend sur toute sa longueur à l'intérieur d'un corps (440), au moins un constituant solide (410, 420) fondant sous l'effet d'un chauffage;
le chauffage du ou des constituants solides (410, 420) introduits, afin de former une matrice plastifiée;
le déplacement de la matrice plastifiée vers un orifice de sortie (450) du canal d'extrusion (430) par l'application d'un travail mécanique sur ladite matrice;
l'extrusion de la matrice plastifiée à travers un orifice de sortie (450) pour former au moins une fibre plastifiée (460);
la structuration d'une ou plusieurs fibres plastifiées (460) en un réseau structurel tridimensionnel, par le dépôt de ladite ou desdites fibres (460) le long d'un trajet défini par le mouvement d'un plateau mobile (480); et
la solidification du réseau structurel tridimensionnel formé par le dépôt d'une ou plusieurs fibres;
le réseau structurel tridimensionnel ainsi déposé étant solidifié par un flux gazeux traversant la structure fibreuse, sous l'effet d'un gradient de pression du gaz à travers ladite structure.

5. Un appareil (500) destiné à la fabrication de formes galéniques pharmaceutiques solides, comprenant:
un boîtier (540) creux, doté d'une surface interne qui délimite un canal d'extrusion (530) présentant une première extrémité (545) et une seconde extrémité (550), ainsi qu'une section transversale s'étendant axialement sur toute sa longueur, depuis ladite première extrémité (545) jusqu'à ladite seconde extrémité (550), et aboutissant à un orifice de sortie (550) au niveau de la seconde extrémité (550);
ledit boîtier (540) comportant au moins un orifice d'alimentation (555), situé entre la première extrémité (545) et la seconde extrémité (550), permettant l'injection d'une matrice plastifiée dans le canal d'extrusion (530);
ledit appareil comprenant en outre au moins un élément de transport (570) servant à extruder la matrice plastifiée présente dans le canal d'extrusion (530) à travers l'orifice de sortie (550), afin de former au moins une fibre plastifiée (560); et
une unité de structuration de fibres comprenant un plateau mobile (580) destiné à structurer une ou plusieurs fibres extrudées (560) sous forme de réseau structurel tridimensionnel, par le dépôt de ladite ou desdites fibres (560) le long d'un trajet défini par le mouvement dudit plateau (580);
ledit plateau, sur lequel sont déposées la ou les fibres, comprenant une grille qui définit une surface de dépôt.

6. Procédé de fabrication de formes galéniques solides, comprenant les étapes consistant à:
injecter une matrice plastifiée dans un canal d'extrusion (530) présentant une section transversale qui s'étend sur toute sa longueur à l'intérieur d'un corps (540);
acheminer la matrice plastifiée vers un orifice de sortie (550) du canal d'extrusion (530) en exerçant une action mécanique sur ladite matrice;
extruder la matrice plastifiée à travers un orifice de sortie (550) pour former au moins une fibre plastifiée (560);
structurer une ou plusieurs fibres plastifiées extrudées (560) sous forme de réseau structurel tridimensionnel, en déposant ladite ou lesdites fibres (560) le long d'un trajet défini par le déplacement d'un plateau mobile (580); et
solidifier le réseau structurel tridimensionnel formé par la ou les fibres déposées;
le réseau structurel tridimensionnel formé par la ou les fibres déposées étant solidifié par un flux de gaz traversant la structure fibreuse, sous l'effet d'un gradient de pression du gaz à travers ladite structure.

7. Dispositif selon l'une quelconque des revendications précédentes, dans lequel la section transversale du canal d'extrusion (330, 430, 530) se réduit, avant un orifice de sortie (350, 450, 550), pour atteindre la section transversale dudit orifice de sortie (350, 450, 550); et/ou dispositif selon l'une quelconque des revendications précédentes, dans lequel le canal d'extrusion (330, 430, 530) se ramifie vers au moins une autre extrémité comprenant un orifice de sortie; et/ou dispositif selon l'une quelconque des revendications précédentes, comprenant en outre au moins une unité d'alimentation en solvant (386) reliée au moins au deuxième orifice d'alimentation (356) pour injecter au moins un solvant dans le canal d'extrusion (330); et/ou dispositif selon l'une quelconque des revendications précédentes, comprenant en outre une unité d'alimentation en solides granulaires (385, 485) pour injecter un ou plusieurs constituants solides granulaires, par l'intermédiaire du premier orifice d'alimentation (355, 455), dans le canal d'extrusion (330, 430).

8. Procédé selon la revendication 2, dans lequel l'injection d'au moins un solvant dans le canal d'extrusion (330) est effectuée au moyen d'une unité d'alimentation en solvant (386) reliée à au moins un deuxième orifice d'alimentation (356) du canal d'extrusion (330).

9. Procédé selon l'une quelconque des revendications précédentes, dans lequel au moins l'un des un ou plusieurs constituants solides injectés (310, 320, 410, 420) est un constituant solide granulaire, et dans lequel ledit constituant solide granulaire est injecté dans le canal d'extrusion (330, 430) au moyen d'une unité d'alimentation en solides granulaires (385, 485).

10. Procédé selon l'une quelconque des revendications précédentes, dans lequel l'acheminement de la matrice plastifiée vers un orifice de sortie (350, 450, 550) du canal d'extrusion (330, 430, 530) est effectué au moyen d'un élément d'acheminement (370, 470, 570) choisi dans le groupe constitué par les vis ou les pistons.

11. Procédé selon l'une quelconque des revendications précédentes, dans lequel le support comprend un support x-y-z; et/ou procédé selon l'une quelconque des revendications précédentes, dans lequel le support comprend une grille définissant une surface de dépôt; et/ou procédé selon l'une quelconque des revendications précédentes, dans lequel le support comprend une grille définissant une surface de dépôt qui est imperméable à une fibre déposée mais qui permet le passage d'un flux d'air ou de gaz; et/ou procédé selon l'une quelconque des revendications précédentes, dans lequel le support comprend une grille à travers laquelle circule un gaz destiné à solidifier un réseau structurel tridimensionnel de fibres déposées; et/ou procédé selon l'une quelconque des revendications précédentes, dans lequel le support comprend une grille à travers laquelle circule un gaz destiné à éliminer le solvant d'un réseau structurel tridimensionnel de fibres déposées; et/ou procédé selon l'une quelconque des revendications précédentes, dans lequel le support comprend une grille à travers laquelle circule un gaz destiné à favoriser la solidification d'un réseau structurel tridimensionnel de fibres déposées, par évaporation du solvant ou par refroidissement.

12. Dispositif selon l'une quelconque des revendications précédentes, dans lequel la platine comprend une platine x-y-z; et/ou dispositif selon l'une quelconque des revendications précédentes, dans lequel la grille définit une surface de dépôt qui est imperméable à une fibre déposée mais qui permet le passage d'un flux d'air ou de gaz; et/ou dispositif selon l'une quelconque des revendications précédentes, dans lequel un gaz peut circuler à travers la grille pour solidifier un réseau structurel tridimensionnel de fibres déposées; et/ou dispositif selon l'une quelconque des revendications précédentes, dans lequel un gaz peut circuler à travers la grille pour éliminer le solvant d'un réseau structurel tridimensionnel de fibres déposées; et/ou dispositif selon l'une quelconque des revendications précédentes, dans lequel un gaz peut circuler à travers la grille pour favoriser la solidification d'un réseau structurel tridimensionnel de fibres déposées, par évaporation du solvant ou par refroidissement.

13. Dispositif selon l'une quelconque des revendications précédentes, dans lequel au moins un élément de transport (370, 470, 570) est choisi dans le groupe constitué par les vis ou les pistons; et/ou dispositif selon l'une quelconque des revendications précédentes, comprenant en outre une unité destinée à souffler du gaz sur une structure déposée; et/ou dispositif selon l'une quelconque des revendications précédentes, dans lequel au moins un orifice de sortie (350, 450, 550) est conçu pour extruder une fibre présentant une épaisseur moyenne inférieure à 2,5 mm.

14. Procédé selon l'une quelconque des revendications précédentes, comprenant en outre un dispositif permettant de souffler du gaz sur une structure déposée.

15. Le procédé selon l'une quelconque des revendications précédentes, dans lequel au moins un orifice de sortie (350, 450, 550) est conçu pour extruder une fibre ayant une épaisseur moyenne de fibre inférieure à 2,5 mm.

16. Procédé selon l'une quelconque des revendications précédentes, dans lequel le réseau structurel tridimensionnel solide de fibres (110, 120, 130, 140, 150, 160, 170, 180) comprend au moins un contact ponctuel entre fibres, ledit contact ponctuel entre fibres présentant une largeur de contact inférieure ou égale à 2 mm; et/ou procédé selon l'une quelconque des revendications précédentes, dans lequel le réseau structurel tridimensionnel solide de fibres (110, 120, 130, 140, 150, 160, 170, 180) comprend au moins un contact ponctuel entre fibres, et dans lequel le nombre desdits contacts ponctuels entre fibres dans le réseau structurel tridimensionnel de fibres (110, 120, 130, 140, 150, 160, 170, 180) est contrôlé avec précision.

17. Procédé selon l'une quelconque des revendications précédentes, dans lequel l'épaisseur d'une fibre et l'espacement entre les fibres au sein du réseau structurel tridimensionnel solide de fibres (110, 120, 130, 140, 150, 160, 170, 180) sont contrôlés avec précision; et/ou procédé selon l'une quelconque des revendications précédentes, dans lequel le réseau structurel tridimensionnel de fibres (110, 120, 130, 140, 150, 160, 170, 180) comprend une structure ordonnée ou partiellement ordonnée ; et/ou le procédé selon l'une quelconque des revendications précédentes, dans lequel la forme galénique solide pharmaceutique comprend un solide contenant un médicament (101) présentant une surface externe (102) et une structure interne (104) contiguë à ladite surface externe (102) et aboutissant à celle-ci, ladite structure interne (104) comprenant un réseau structurel tridimensionnel constitué d'une ou plusieurs fibres (110, 120, 130, 140, 150, 160, 170, 180), lesdites fibres (110, 120, 130, 140, 150, 160, 170, 180) comprenant au moins un principe actif et au moins un excipient, lesdites fibres (110, 120, 130, 140, 150, 160, 170, 180) comprenant en outre des segments de fibre séparés et espacés des segments de fibre adjacents par des espaces libres, ces espaces libres définissant un ou plusieurs espaces vides (105) au sein du solide contenant un médicament (101), la ou les fibres (110, 120, 130, 140, 150, 160, 170, 180) présentant une épaisseur moyenne comprise entre 1 µm et 2,5 mm, et l'espace libre effectif entre les segments de fibre, à travers le ou les espaces vides (105), étant en moyenne supérieur à 1 µm.
